# EUROPEAN PATENT APPLICATION

(11) **EP 4 530 344 A1**
(43) Date of publication of application: **02.04.2025**
(21) Application number: 23811780.8
(22) Date of filing: 22.05.2023
(51) Int. Cl.: C12N 5/071

(54) **PRODUCTION METHOD FOR RENAL COLLECTING DUCT CELLS AND PELVIC EPITHELIAL CELLS**

(30) Priority: 23.05.2022 JP 2022084048
(71) Applicant: Kyoto University, Kyoto-shi, Kyoto 606-8501 (JP)
(72) Inventor: OSAFUNE, Kenji, Kyoto-shi, Kyoto 606-8501 (JP); RYOSAKA, Makoto, Kyoto-shi, Kyoto 604-0847 (JP); MAE, Shinichi, Kyoto-shi, Kyoto 606-8501 (JP)
(74) Representative: Hoffmann Eitle
(86) International application number: PCT/JP2023/018974
(87) International publication number: WO 2023/228908

(57) **Abstract**

Provided is a method for producing renal collecting duct principal cells, including culturing ureteric bud cells or cells that have been differentiated from ureteric bud cells in a medium comprising a TGFβ inhibitor, a Wnt inhibitor, and a phosphodiesterase inhibitor. Also, provided is a method for producing a renal collecting duct principal cell-containing organoid, including culturing a ureteric bud organoid or an organoid that has been differentiated from a ureteric bud organoid in a medium comprising a TGFβ inhibitor, a Wnt inhibitor, and a phosphodiesterase inhibitor.

## Description

### Technical Field

The present application relates to a method for producing renal collecting duct principal cells, renal collecting duct principal cell-containing organoids, renal collecting duct intercalated cells, or renal pelvic epithelial cells.

### Background Art

The renal collecting duct mainly concentrates and adjusts the pH of the primitive urine that has been filtered by the glomerulus and passed through the renal tubules to maintain the homeostasis of the water content and the acid-base balance in the body. The renal collecting duct is composed of principal cells and intercalated cells (intercalary cells). The principal cells are the main component of the collecting duct that express water channels such as AQP2 and serve to concentrate urine. The intercalated cells express carbonic dehydrogenase and the like and adjust pH. The production of renal collecting duct cells and renal pelvic epithelial cells are expected to be applied to the model production, pathological analysis, and therapy development for ion channel anomalies and congenital anomalies of kidney and urinary tract including cystic kidney disease.

To date, it has been reported that nephron progenitor cells, which are progenitor cells of glomeruli and renal tubules, and ureteric bud cells, which are collecting duct progenitor cells, were induced to differentiate from human pluripotent stem cells, and renal progenitor cells of these two were combined for culture to produce renal tissues comprising a collecting duct in addition to glomeruli and renal tubules (Non Patent Literatures 1 and 2), and it has also been reported that ureteric bud lineage cells derived from human pluripotent stem cells were purified using a flow cytometer, and ureteric bud organoids partially comprising collecting duct cells were produced from the cells thus purified (Non Patent Literature 3). However, there have been no reports on a method for selectively inducing differentiation of renal collecting duct cells and renal pelvic epithelial cells from human pluripotent stem cells.

### Citation List

### Non Patent Literature

Non Patent Literature 1: Tsujimoto H. et al., Cell Reports 2020; 31(1):107476.
Non Patent Literature 2: Uchimura. et al., Cell Reports 2020; 33(11):108514.
Non Patent Literature 3: Zeng. et al., Nature Communications 2021; 12:3641.

### Summary of Invention

### Technical Problem

An object of the present application is to provide a method for producing renal collecting duct principal cells, renal collecting duct principal cell-containing organoids, renal collecting duct intercalated cells, or renal pelvic epithelial cells.

### Solution to Problem

The present application provides a method for producing renal collecting duct principal cells, including culturing ureteric bud cells or cells that have been differentiated from ureteric bud cells in a medium comprising a TGFβ inhibitor, a Wnt inhibitor, and a phosphodiesterase inhibitor.

The present application also provides a cell population comprising 40% or more renal collecting duct principal cells.

The present application also provides a method for producing a renal collecting duct principal cell-containing organoid, including culturing a ureteric bud organoid or an organoid that has been differentiated from a ureteric bud organoid in a medium comprising a TGFβ inhibitor, a Wnt inhibitor, and a phosphodiesterase inhibitor.

The present application also provides a renal collecting duct principal cell-containing organoid comprising 40% or more renal collecting duct principal cells.

The present application also provides a method for producing renal collecting duct intercalated cells, including culturing renal collecting duct principal cells in a medium comprising a TGFβ inhibitor, a Wnt inhibitor, a retinoic acid receptor agonist, and a Notch signal inhibitor, in the presence of an ETS family protein.

The present application also provides a cell population comprising 30% or more renal collecting duct intercalated cells.

The present application also provides a method for producing renal pelvic epithelial cells, including culturing renal collecting duct progenitor cells in a medium comprising a TGFβ inhibitor, a Wnt inhibitor, a retinoic acid receptor agonist, and a Notch signal inhibitor, in the presence of an ETS family protein.

The present application also provides a cell population comprising 40% or more renal pelvic epithelial cells.

### Advantageous Effects of Invention

The methods according to the present application make it possible to produce renal collecting duct principal cells, renal collecting duct principal cell-containing organoids, renal collecting duct intercalated cells, or renal pelvic epithelial cells.

### Brief Description of Drawings

[Figure 1] Figure 1 shows a schematic diagram illustrating a procedure for generating an iPS cell line (Tet-On-ELF5-1231A3) capable of forced expression of ELF5 and performing forced expression of ELF5 in the iPS cells.
[Figure 2] Figure 2 shows an example of a protocol for forced expression of ELF5 in Tet-On-ELF5-1231A3 iPS cells. Tet-On-ELF-1231A3 iPS cells were cultured in StemFit (registered trademark) AK02 for 1 day, and then, 4 µM doxycycline was added thereto to culture for another 1 day.
[Figure 3] Figure 3 shows a comparison of semiquantitative PCR results with or without doxycycline (DOX) treatment.
[Figure 4] Figure 4 shows a bright field image and fluorescence microscopic images of the Tet-On-ELF5-1231A3 iPS cells treated with doxycycline according to the protocol of Figure 2.
[Figure 5] Figure 5 shows immunostaining images of the Tet-On-ELF5-1231A3 iPS cells treated with doxycycline according to the protocol descrbed in Figure 2.
[Figure 6] Figure 6 shows an example of a protocol for inducing mesonephric duct cells from the Tet-On-ELF5-1231A3 iPS cells. Anterior PS: Anterior primitive streak, Anterior IM: Anterior intermediate mesoderm, ND: Mesonephric duct, UB: Ureteric bud, LDN: LDN193189.
[Figure 7] Figure 7 shows immunostaining images of the mesonephric duct cells induced according to the protocol of Figure 6.
[Figure 8] Figure 8 shows an example of a protocol for inducing ureteric bud organoids from anterior intermediate mesoderm cells derived from the Tet-On-ELF5-1231A3 iPS cells. iUB: Ureteric bud.
[Figure 9] Figure 9 shows a bright field image of a ureteric bud organoid on Day 2 induced from the anterior intermediate mesoderm cells according to the protocol of Figure 8.
[Figure 10] Figure 10 shows an example of a protocol for inducing renal collecting duct progenitor cells from the Tet-On-ELF5-1231A3 iPS cells.
[Figure 11] Figure 11 shows immunostaining images of the cells induced according to the protocol of Figure 10.
[Figure 12] Figure 12 shows an example of a protocol for inducing renal collecting duct progenitor cells from the Tet-On-ELF5-1231A3 iPS cells. To a renal collecting duct progenitor cell-induction medium, 4 µM doxycycline and 400 µM IBMX were further added.
[Figure 13] Figure 13 shows immunostaining images of renal collecting duct principal cells induced according to the protocol of Figure 12.
[Figure 14] Figure 14 shows an example of a protocol for inducing renal collecting duct progenitor cells from the Tet-On-ELF5-1231A3 iPS cells. To a renal collecting duct progenitor cell-induction medium, 4 µM doxycycline and IBMX at various concentrations were further added.
[Figure 15] Figure 15 shows an immunostaining image of renal collecting duct principal cells induced according to the protocol of Figure 14.
[Figure 16] Figure 16 shows an example of a protocol in which renal collecting duct principal cells were subjected to transwell culture from mature mesonephric duct cell aggregates derived from the Tet-On-ELF5-1231A3 iPS cells, and desmopressin (dDAVP) was added thereto. CD: Renal collecting duct, PC: Principal cell.
[Figure 17] Figure 17 shows a schematic diagram of transwell culture.
[Figure 18] Figure 18 shows a comparison of AQP2 expression and polarity by immunostaining of renal collecting duct principal cells induced according to the protocol of Figure 16.
[Figure 19] Figure 19 shows an example of a protocol for inducing renal collecting duct principal cells from mature mesonephric duct cell aggregates derived from the Tet-On-ELF5-1231A3 iPS cells. Various factors were added to a renal collecting duct principal cell-induction medium excluding IBMX, and renal collecting duct principal cells were induced by planar culture.
[Figure 20] Figure 20 shows immunostaining images of the renal collecting duct principal cells induced according to the protocol of Figure 19, with E-CADHERIN (red), AQP2 (white), and AVPR2 (green). AVP: Vasopressin acetate, ALD: Aldosterone.
[Figure 21] Figure 21 shows AQP2-positive cell rates in the renal collecting duct principal cells induced according to the protocol of Figure 19. 2F: Aldosterone + K252a.
[Figure 22] Figure 22 shows an example of a protocol for inducing renal collecting duct principal cells from mature mesonephric duct cell aggregates derived from the Tet-On-ELF5-1231A3 iPS cells. To the renal collecting duct principal cell-induction medium, 10 nM aldosterone, 0.1 µM TTNPB, and 100 nM dDAVP were further added.
[Figure 23] Figure 23 shows immunostaining images of the renal collecting duct principal cells induced according to the protocol of Figure 22.
[Figure 24] Figure 24 shows an example of a protocol for inducing renal collecting duct principal cells from ureteric bud organoids derived from the Tet-On-ELF5-1231A3 iPS cells.

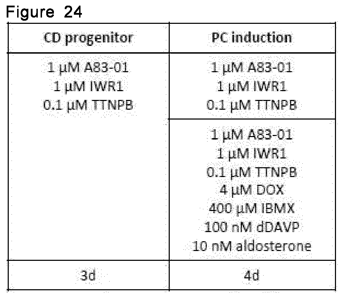

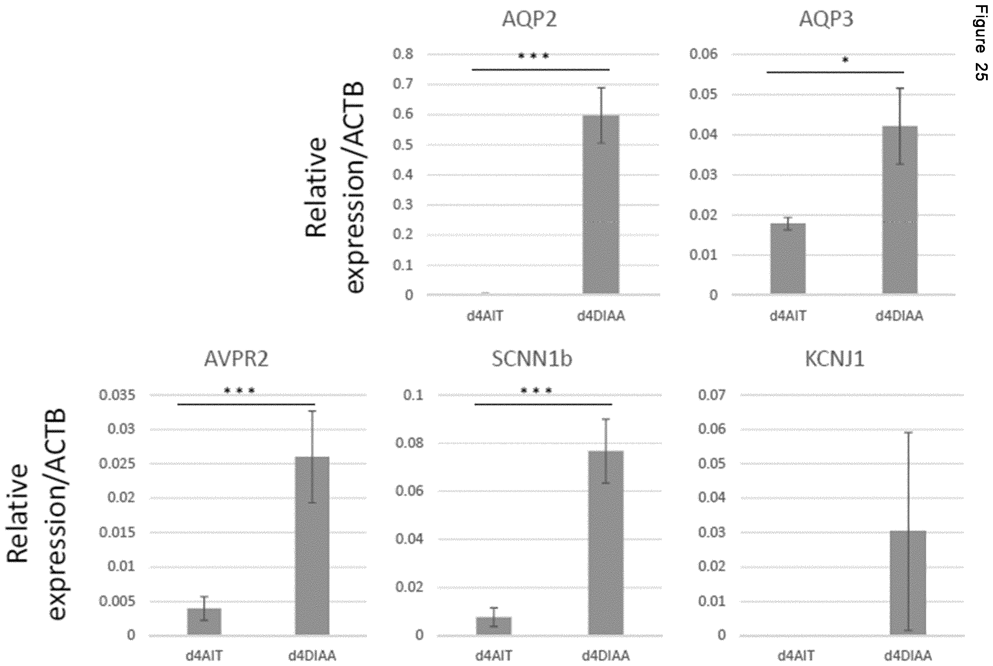

[Figure 25] Figure 25 shows a comparison of expression levels of renal collecting duct principal cell-related marker genes in collecting duct principal cells induced according to the protocol of Figure 24. d4AIT: Cells obtained by culturing ureteric bud cells in a renal collecting duct progenitor cell-induction medium comprising TTNPB for 7 days, d4DIAA: Renal collecting duct principal cells induced by culturing ureteric bud cells in a renal collecting duct progenitor cell-induction medium comprising TTNPB for 3 days, and further in a renal collecting duct principal cell-induction medium comprising TTNPB, dDAVP, and aldosterone for 4 days.
[Figure 26] Figure 26 shows an example of a protocol for inducing renal collecting duct principal cell-containing organoids from mature mesonephric duct cell aggregates derived from the Tet-On-ELF5-1231A3 iPS cells.
[Figure 27] Figure 27 shows a bright field image of a ureteric bud organoid on Day 6, and a bright field image and a fluorescence microscopic image of a renal collecting duct principal cell-containing organoid on Day 2, induced according to the protocol of Figure 26.
[Figure 28] Figure 28 shows a bright field image of a renal collecting duct principal cell-containing organoid on Day 6 induced according to the protocol of Figure 26.
[Figure 29] Figure 29 shows immunostaining images of a renal collecting duct principal cell-containing organoid induced according to the protocol of Figure 26.
[Figure 30] Figure 30 shows diagrams to illustrate the functional evaluation tests of the renal collecting duct principal cell-containing organoids. dDAVP alone or dDAVP and tolvaptan was added to the renal collecting duct principal cell-containing organoid on Day 6 of the induction, which was further cultured. Bright field images of the renal collecting duct principal cell-containing organoid on Day 1 and Day 3 of culture are shown. Arrowheads: Areas of cystic changes due to water uptake.
[Figure 31] Figure 31 shows a schematic diagram illustrating a procedure for producing a PKD1 knockout iPS cell line (Tet-On-ELF5-27B6-5) capable of forced expression of ELF5 and performing forced expression of ELF5 in the iPS cells.
[Figure 32] Figure 32 shows an example of a protocol for forced expression of ELF5 in the Tet-On-ELF5-27B6-5 iPS cells. The Tet-On-ELF5-27B6-5 iPS cells were cultured in StemFit (registered trademark) AK02 for 1 day, and then, 4 µM doxycycline was added thereto to culture for another 1 day.
[Figure 33] Figure 33 shows a bright field image and immunostaining images of the Tet-On-ELF5-27B6-5 iPS cells treated with doxycycline according to the protocol of Figure 32.
[Figure 34] Figure 34 shows immunostaining images of the Tet-On-ELF5-27B6-5 iPS cells treated with doxycycline according to the protocol of Figure 32.
[Figure 35] Figure 35 shows an example of a protocol for inducing ureteric bud organoids from the Tet-On-ELF5-27B6-5 iPS cells.
[Figure 36] Figure 36 shows bright field images of the ureteric bud organoid induced according to the protocol of Figure 35.
[Figure 37] Figure 37 shows TEER measurement results of renal collecting duct principal cells before the administration of benzamil (pre) and 24 hours after the administration of benzamil (24h). A group that did not receive benzamil was used as a control (Ctl).
[Figure 38] Figure 38 shows immunostaining images of renal collecting duct principal cells that have been treated with or without benzamil.
[Figure 39] Figure 39 shows a comparison of ENaC-positive cell rates in renal collecting duct principal cells with and without the administration of benzamil (Bz). A group that did not receive benzamil was used as a control (Ctl). Student's t-test was used to test for significant differences. N.S.: No significant differences.
[Figure 40] Figure 40 shows a schematic diagram illustrating the induction of renal collecting duct intercalated cells (IC) from ureteric bud organoids (iUB) through renal collecting duct progenitor cells (CDP) and renal collecting duct principal cells (PC). DOX: Doxycycline.
[Figure 41] Figure 41 shows an example of a protocol for inducing renal collecting duct intercalated cells from ureteric bud cells derived from the Tet-On-ELF5-1231A3 iPS cells. IC: Intercalated cells.
[Figure 42] Figure 42 shows immunostaining images of the renal collecting duct intercalated cells induced according to the protocol of Figure 41.
[Figure 43] Figure 43 shows a schematic diagram illustrating the induction of renal pelvic epithelial cells from ureteric bud organoids (iUB) through renal collecting duct progenitor cells (CDP).
[Figure 44] Figure 44 shows an example of a protocol for inducing renal pelvic epithelial cells from ureteric bud cells derived from the Tet-On-ELF5-1231A3 iPS cells.
[Figure 45] Figure 45 shows immunostaining images of the renal pelvic epithelial cells induced according to the protocol of Figure 44.
[Figure 46] Figure 46 shows an example of a protocol for inducing renal collecting duct principal cells from ureteric bud cells derived from the Tet-On-ELF5-1231A3 iPS cells.
[Figure 47] Figure 47 shows immunostaining images of the renal collecting duct principal cells induced according to the protocol of Figure 46.

### Description of Embodiments

In the present disclosure, when a numerical value is accompanied by the term "about", it is intended to include a range of ± 10% of that value. For example, "about 20" includes "18 to 22". The numerical range includes all numbers between and including the endpoints. The "about" relating to a range applies to the endpoints of that range. For example, "about 20 to 30" includes "18 to 33".

In the specification and claims of the present application, the expression "a certain type of cells" means a cell group in which the specified type of cells are included, unless otherwise stated, and cells of types other than those of the specified types may be included in the cell group. For example, the expression "a culture of a certain type of cells" means a culture of a cell group in which the specified type of cells are included, and cells other than those of the specified types may be included. In a similar manner, the expression "a certain type of cell population" means a cell population in which the specified type of cells are included, unless otherwise stated, and cells other than those of the specified types may be included in the cell population.

### Method for Producing Renal Collecting Duct Principal Cells

An aspect of the present application provides a method for producing renal collecting duct principal cells, including culturing ureteric bud cells or cells that have been differentiated from ureteric bud cells in a medium comprising a TGFβ inhibitor, a Wnt inhibitor, and a phosphodiesterase inhibitor.

In the present application, whether the cells are renal collecting duct principal cells may be confirmed by the expression of markers, such as AQP2, AQP3, AQP4, AVPR2, SCNN1b, ELF5, and KCNJ1. Optionally, it may be confirmed that carbonic anhydrase (CA) II, which is a marker for renal collecting duct intercalated cells, is not expressed. It may be visually confirmed that the renal collecting duct principal cells express a renal collecting duct principal cell marker, under a microscope by immunostaining, or it may be confirmed by a flow cytometry, for example, fluorescence-activated cell sorting (FACS).

In the present application, animals from which ureteric bud cells are derived are not limited, and examples thereof include mammals such as a mouse, a rat, a hamster, a guinea pig, a cow, a horse, a pig, a sheep, a monkey, an orangutan, a chimpanzee, a dog, a cat, a bird, and a human, preferably primates, and more preferably a human. Whether the cells are ureteric bud cells may be confirmed by the expression of markers, such as GATA3, RET, WNT11, LHX1, SOX9, and a very low density lipoprotein receptor (VLDL-R), uptake of a very low density lipoprotein (VLDL), or the like. In an embodiment, ureteric bud cells are cultured in a medium comprising a TGFβ inhibitor, a Wnt inhibitor, and a phosphodiesterase inhibitor.

Cells differentiated from ureteric bud cells may be any cells generated before differentiating into renal collecting duct principal cells, and for example, may be renal collecting duct progenitor cells. The renal collecting duct progenitor cells refer to cells that express aquaporin 2 (AQP2), but do not express other collecting duct principal cell marker genes such as an arginine vasopressin receptor 2 (AVPR2). Whether the cells are renal collecting duct progenitor cells may be confirmed by the expression of AQP2, and without the expression of AVPR2. In one embodiment, the renal collecting duct progenitor cells differentiated from the ureteric bud cells are cultured in a medium comprising a TGFβ inhibitor, a Wnt inhibitor, and a phosphodiesterase inhibitor.

The ureteric bud cells may be those induced to differentiate from pluripotent stem cells. For example, the ureteric bud cells may be obtained by dissociating ureteric bud organoids induced to differentiate from pluripotent stem cells, into single cells. Methods for inducing ureteric bud organoids to differentiate from pluripotent stem cells are known, and any known method can be used. For example, the method described in WO2019/098349, or in Mae, SI. & Ryosaka, M. et al., Cell Reports 32, 4, 107943 may be used. In particular, a method for inducing ureteric bud organoids to differentiate from pluripotent stem cells including the following steps may be used:
(i) culturing pluripotent stem cells in the presence of an activator of activin receptor kinase 4,7 and a GSK3β inhibitor to obtain an anterior primitive streak cell culture;
(ii) culturing the anterior primitive streak cell culture in the presence of a fibroblast growth factor, a retinoic acid receptor agonist, a TGFβ inhibitor, and a BMP inhibitor to obtain an anterior intermediate mesoderm cell culture;
(iii) culturing the anterior intermediate mesoderm cell culture in the presence of a GSK3β inhibitor, a BMP inhibitor, a fibroblast growth factor, a retinoic acid receptor agonist, and a glial cell line-derived neurotrophic factor to obtain an early mesonephric duct (Wolffian duct) cell culture;
(iv) culturing the early mesonephric duct (Wolffian duct) cells in the presence of a GSK3β inhibitor, a BMP inhibitor, a fibroblast growth factor, a retinoic acid receptor agonist, and a glial cell line-derived neurotrophic factor to obtain a mature mesonephric duct (Wolffian duct) cell aggregate; and
(v) culturing the mature mesonephric duct (Wolffian duct) cell aggregate in the presence of a GSK3β inhibitor, a BMP inhibitor, a fibroblast growth factor, a retinoic acid receptor agonist, a glial cell line-derived neurotrophic factor, and a cell culture substrate (e.g., Matrigel) to obtain a ureteric bud organoid.

The expression "pluripotent stem cell" in the present application refers to a stem cell that has both pluripotency capable of differentiating into any cell existing in a living body, and proliferative capacity, and examples thereof include embryonic stem (ES) cells (J.A. Thomson et al. (1998), Science 282:1145-1147; J.A. Thomson et al. (1995), Proc. Natl. Acad. Sci. USA, 92:7844-7848;J.A. Thomson et al. (1996), Biol. Reprod., 55:254-259; J.A. Thomson and V.S. Marshall (1998), Curr. Top. Dev. Biol., 38:133-165), embryonic stem (ntES) cells derived from cloned embryos obtained by nuclear transfer (T. Wakayama et al., (2001), Science, 292:740-743; S. Wakayama et al., (2005), Biol. Reprod., 72:932-936; J. Byrne et al., (2007), Nature, 450:497-502), sperm stem cells ("GS cells") (M. Kanatsu-Shinohara et al., (2003) Biol. Reprod., 69:612-616; K. Shinohara et al., (2004), Cell, 119:1001-1012), embryonic germ cells ("EG cells") (Y. Matsui et al., (1992), Cell, 70:841-847; J. L. Resnick et al., (1992), Nature, 359:550-551), induced pluripotent stem (iPS) cells (K. Takahashi and S. Yamanaka (2006) Cell, 126:663-676; K. Takahashi et al. (2007), Cell, 131:861-872; J. Yu et al. (2007), Science, 318:1917-1920; Nakagawa, M. et al, Nat. Biotechnol. 26:101-106 (2008);WO2007/069666), and pluripotent cells derived from cultured fibroblasts or myeloid stem cells (Muse cells) (WO2011/007900). Animals from which pluripotent stem cells are derived are not limited, and examples thereof include mammals such as a mouse, a rat, a hamster, a guinea pig, a cow, a horse, a pig, a sheep, a monkey, an orangutan, a chimpanzee, a dog, a cat, a bird, and a human, preferably primates, and more preferably a human. Also, the pluripotent stem cells may be ES cells or iPS cells, and for example, they are iPS cells.

The iPS cells may be produced by introducing specific reprogramming factors in the form of DNA or a protein into somatic cells (K. Takahashi and S. Yamanaka (2006) Cell, 126:663-676; K. Takahashi et al. (2007), Cell, 131:861-872; J. Yu et al. (2007), Science, 318:1917-1920; Nakagawa, M. et al, Nat. Biotechnol. 26:101-106 (2008); WO2007/069666). When iPS cells are used, the iPS cells may be produced from somatic cells by a method known per se, or iPS cells that have already been established and stocked may be used. Somatic cells from which the iPS cells used in the present invention are derived are not limited, and for example, peripheral blood-derived cells or cord blood-derived cells may be used.

In the present application, a medium may be prepared by appropriately adding necessary factors to a basal medium used for culturing animal cells. Examples of the basal media include MEM zinc option medium, IMEM zinc option medium, IMDM medium, Medium 199 medium, Eagle's minimum essential medium (EMEM) medium, αMEM medium, Dulbecco's modified Eagle's medium (DMEM) medium, DMEM/F12 medium, Ham's F12 medium, RPMI 1640 medium, Fischer's medium, and a mixed medium thereof. The basal medium may contain serum (e.g., fetal bovine serum (FBS)), or may be serum-free. The basal medium may contain one or more serum alternatives such as albumin, transferrin, KnockOut Serum Replacement (KSR) (serum alternative in ES cell culture) (Thermo Fisher Scientific, Inc, N2 supplement (Thermo Fisher Scientific, Inc.), B27 supplement (Thermo Fisher Scientific, Inc.), fatty acids, insulin, collagen precursors, microelements, 2-mercaptoethanol, and 3'-thiol glycerol, one or more substances such as lipids, amino acids, L-glutamine, GlutaMax (Thermo Fisher Scientific, Inc.), non-essential amino acids (NEAA), vitamins, growth factors, antibiotics, antioxidants, pyruvic acid, buffer agents, inorganic salts, and equivalents thereof, or one or more other substances to be generally added to a medium for animal culture, as needed. The basal medium used in the present application may be, for example, DMEM/F12 medium.

In an embodiment, the ureteric bud cells or the cells that have been differentiated from the ureteric bud cells are cultured in the presence of an ETS family protein. Examples of the ETS family include ELF (ELF1, ELF2, ELF3, ELF4, and ELF5), EHF, GABPA, ERG, FLI1, FEV, ERF, ELK1, ELK3, ELK4, ETS1, ETS2, ETV1, ETV2, ETV3, ETV4, ETV5, ETV6, ETV7, SPDEF, SPI1, SPIB, and SPIC. In the present application, the ETS family gene may be appropriately selected from those known above; however, it is, for example, ELF, and preferably ELF5. Examples of ELF5 include proteins comprising the amino acid sequences shown in NCBI Reference Sequence: NP_001230009.1, NP_001230010.1, NP_001413.1, NP_938195.1, XP_016872797.1, or XP_016872798.1. In an embodiment, a protein that comprises an amino acid sequence in which one or more amino acids have been substituted, deleted, added and/or inserted may be used as ELF5, in the amino acid sequences shown in NCBI Reference Sequence: NP_001230009.1, NP_001230010.1, NP_001413.1, NP_938195.1, XP_016872797.1, or XP_016872798.1, as long as the production of renal collecting duct principal cells are possible. The phrase "one or more" in the present embodiment means preferably 1 to 20, and more preferably 1 to 15, for example, 1, 2, 3, 4, 5, 6, 7, 8, 9, or 10. In another embodiment, a protein that comprises an amino acid sequence having a sequence identity of about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% or more with the amino acid sequences shown in NCBI Reference Sequence: NP_001230009.1, NP_001230010.1, NP_001413.1, NP_938195.1, XP_016872797.1, or XP_016872798.1 may be used as ELF5, as long as the production of renal collecting duct principal cells are possible.

The substitutions of amino acids described above may be conservative amino acid substitutions, or non-conservative amino acid substitutions. The conservative amino acid substitutions used herein are those that may be generally obtained without altering physiological activity of the resulting molecules, in other words, those recognized within the conservative substitutions (e.g., Watson et al., Molecular Biology of the Gene). Examples thereof may include substitutions that occur between amino acids which have similarity of their side-chains, such as aspartic acid and glutamic acid (acidic amino acids); lysine, arginine and histidine (basic amino acids); alanine, valine, leucine, isoleucine, proline, phenylalanine, methionine, and tryptophan (non-polar amino acids); glycine, asparagine, glutamine, cysteine, serine, threonine, and tyrosine (uncharged polar amino acids); and phenylalanine, tryptophan, and tyrosine (aromatic amino acids). Similarly, the amino acids may be classified as follows: glycine, alanine, valine, leucine, isoleucine, serine, and threonine (aliphatic amino acids); serine and threonine (aliphatic-hydroxy amino acids); asparagine and glutamine (amide-type amino acids); and cysteine and methionine (sulfur-comprising amino acids).

The term "sequence identity" used herein refers to a percentage of identical bases or amino acids at corresponding positions in two or more sequences (nucleotide sequences or amino acid sequences), when the two or more sequences are aligned in consideration of gaps and insertion so that the degree of sequence matching becomes maximum. Methods of determining the identity are designed to confer a maximum degree of matching between aligned sequences. Examples of the method of determining the identity between two sequences include, but are not limited to, BLASTP, BLASTN, and FASTA. Also, DNASIS (manufactured by Hitachi Software Engineering Co., Ltd.), or GENETYX (manufactured by Nippon Genetics Co., Ltd.) may be used for determination. Alternatively, sequences may be simply compared for determination in case of short peptides. Those skilled in the art may determine identity between sequences using methods such as those described above.

The ETS family protein used in the present application may be one in which its signal peptide sequence has been substituted for any signal peptide sequence. A tag sequence such as a FLAG tag, a HA tag, a His tag, a Myc tag, and a V5 tag may be added to the ETS family protein used in the present application. The ETS family protein used in the present application may be a protein derived from a human, or a protein derived from other mammals (e.g., a mouse, a rat, a cow, a horse, a pig, a sheep, a monkey, a dog, a cat, and a bird) (i.e., ortholog).

The ETS family protein may be added in a medium, or ureteric bud cells or cells that have been differentiated from ureteric bud cells may express the ETS family protein. When the ETS family protein is added in a medium, the concentration of the ETS family protein may be appropriately selected by those skilled in the art according to the ETS family protein to be used. When the ETS family protein is ELF5, its concentration is, for example, 1 nM to 1 mM.

In an embodiment, the ureteric bud cells or the cells that have been differentiated from the ureteric bud cells express an ETS family protein. In the present application, the ureteric bud cells that express the ETS family protein or cells that express the ETS family protein and have been differentiated from the ureteric bud cells only need to express the ETS family protein, and for example, they are cells in which an ETS family gene has been introduced. As a means of introducing genes, known methods can be used, and examples thereof include an infection technique by using a viral vector (e.g., retroviral vector, lentiviral vector, Sendai virus vector, adenoviral vector, or adeno-associated viral vector), a gene transfer technique (e.g., calcium phosphate method, lipofection method, RetroNectin method, or electroporation method) by using a plasmid vector (e.g., plasmid vector, transposon vector, or episomal vector), a gene transfer technique (e.g., calcium phosphate method, lipofection method, or electroporation method) by using an RNA vector, a protein direct infusion method (e.g., a method by using a needle, lipofection method, or electroporation method). Preferably, introduction is performed by a method without integration into the genome of the cell, or a method capable of easily removing from the genome. For this purpose, it is preferable to use PiggyBac (trademark) vector, which is an episomal vector or transposon vector.

In the present application, the ETS family gene is not particularly limited as long as it is a gene that encodes the ETS family protein described above, and for example, it is ELF5 gene. Examples of the nucleotide sequence that encodes the ELF5 gene include the nucleotide sequences of NCBI Reference Sequence: NM_001243080.2, NM_001243081.2, NM_001422.4, NM_198381.2, XM_017017308.1, or XM_017017309.1.

In an embodiment, the ureteric bud cells that express a ETS family protein or the cells that have been differentiated from the ureteric bud cells expressing a ETS family protein may transiently express the ETS family protein. The transient expression of the ETS family protein may be performed using a system that may control on and off of the gene expression. Examples of such a system include one in which the expression is reversibly controlled due to the presence or absence of an agent. Examples thereof include a tetracycline (Tet) expression induction system, a Cumate repressor protein CymR system, and a coumermycin/novobiocin-regulated system.

Examples of the tetracycline (Tet) expression induction system include the Tet-On (registered trademark)/Tet-Off (registered trademark) Gene Expression Systems (Clontech Laboratories, Inc.). In the Tet-on (registered trademark) system, Tet-on (registered trademark) regulatory plasmid that expresses reverse tetracycline-controlled transactivator (rtTA) and a plasmid that encodes tetracycline response element (TRE) having tetO repetitive sequences are used. The rtTA is a fusion protein that is composed of mutant Tet repressor protein (rTetR) and VP16 activation domain (AD), and it binds with tetracycline response element (TRE) by adding doxycycline (Dox) in the medium to induce downstream gene expression. In the Tet-Off (registered trademark) system, gene expression is induced in the absence of Dox.

The Cumate repressor protein CymR system is a system that can reversibly induce the expression of a gene of interest using a lentiviral vector, and it can introduce an expression vector for the gene of interest and an expression vector for CymR repressor into cells to perform the induction by adding an expression inducer Cumate. As the Cumate repressor protein CymR system, SparQ Cumate Switch Inducible System (System Biosciences, LLC) may be used.

As the coumermycin/novobiocin-regulated system, Regulated Mammalian Expression System (Promega Corporation) may be used.

The tetracycline (Tet) expression induction system can be preferably used, among systems that can control on and off of the expression of the genes described above. In an embodiment, the transient expression of the ETS family protein can be controlled by the tetracycline expression induction system, and the medium further comprises doxycycline. Doxycycline can be commercially available, for example, from LKT Laboratories, Inc. In the present embodiment, the concentration of doxycycline may be 4 nM to 400 µmM, 40 nM to 400 µM, or 400 nM to 40 µM, and it is, for example, about 4 µM.

The introduced ETS family gene may be removed from cells when expression is no longer necessary. When the gene transfer vector is an episomal vector, the vector spontaneously disappears after culturing the cells for long term. When a PiggyBac vector is used, the gene that was once integrated into the genome can be removed by PiggyBac (trademark) transposase.

In an embodiment, the ureteric bud cells may be those induced from pluripotent stem cells that express the ETS family protein. The pluripotent stem cells that express the ETS family protein may be any pluripotent stem cells expressing the ETS family protein, and for example, they are pluripotent stem cells in which the ETS family gene has been introduced. The procedures to introduce a gene are described above.

In an embodiment, the pluripotent stem cells that express the ETS family protein may transiently express the ETS family protein. The transient expression of the ETS family protein may be performed using the methods described above. In particular, the tetracycline (Tet) gene expression induction system may be preferably used. For example, the transient expression of the ETS family protein may be controlled by the tetracycline expression induction system.

The "TGFβ inhibitor" is a substance for inhibiting signaling from the bonding of TGFβ to a receptor through SMAD, and is not particularly limited as long as it is a substance for inhibiting the bonding to an ALK family, which is a receptor, or a substance for inhibiting phosphorylation of SMAD by the ALK family. Examples thereof include Lefty-1 (e.g., NCBI Accession Nos.: NM_010094 for mouse, and NM_020997 for human), SB431542, SB202190 (R. K. Lindemann et al., Mol. Cancer, 2003, 2:20), SB505124 (GlaxoSmithKline plc), NPC30345, SD093, SD908, SD208 (Scios, Inc.), LY2109761, LY364947, LY580276 (Lilly Research Laboratories, Inc.), A83-01 (3-(6-methyl-2-pyridinyl)-N-phenyl-4-(4-quinolinyl)-1H-pyrazole-1-carbothioamide, WO2009146408), an ALK5 inhibitor II (2-[3-[6-methylpyridin-2-yl]-1H-pyrazol-4-yl]-1,5-naphthyridine), a TGFβRI kinase inhibitor VIII (6-[2-tert-butyl-5-[6-methyl-pyridin-2-yl]-1H-imidazol-4-yl]-quinoxaline), and derivatives thereof. The TGFβ inhibitor used in the present application may be, for example, A83-01. In this embodiment, the concentration of the TGFβ inhibitor can be appropriately selected by those skilled in the art according to the TGFβ inhibitor to be used. When the TGFβ inhibitor is A83-01, its concentration may be 1 nM to 100 µM, 10 nM to 100 µM, or 100 nM to 10 µM, and it is, for example, about 1 µM.

The "Wnt inhibitor" is not particularly limited as long as it is a substance for inhibiting the signaling pathway through Wnt, and examples thereof include IWR-1, IWP-2, IWP-3, IWP-4, 2-(4-trifluoromethylphenyl)-7,8-dihydro-5H-thiopyrano[4,3-d]pyrimidin-4(3H)-one (XAV939), G-CSF, IGFBP4, Dkk1, Cerberus, an anti-Wnt antibody, a Wnt antagonist (Wnt receptor inhibitor), a soluble Wnt receptor protein (e.g., Frzb-1), and a dominant negative product. The Wnt inhibitor used in the present application may be, for example, IWR-1. In this embodiment, the concentration of the Wnt inhibitor can be appropriately selected by those skilled in the art according to the Wnt inhibitor to be used. When the Wnt inhibitor is IWR-1, its concentration may be 1 nM to 100 µM, 10 nM to 100 µM, or 100 nM to 10 µM, and it is, for example, about 1 µM.

The "phosphodiesterase (PDE) inhibitor" is not particularly limited as long as it is a substance for inhibiting an enzyme that hydrolyzes cyclic phosphodiester such as cAMP and cGMP, and examples thereof include IBMX (3-isobutyl-1-methylxanthine), caffeine, theophylline, theobromine, pentoxifylline, 8-methoxy-3-isobutyl-1-methylxanthine, erythro-9-(2-hydroxy-3-nonyl)adenine hydrochloride (EHNA hydrochloride), cilostamide, milrinone, trexine, etazolate hydrochloride, denbufylline, vardenafil, sildenafil, zaprinast, tadalafil, dipyridamole, 4-{[3',4'-(methylenedioxy)benzyl]amino}-6-methoxyquinazoline, 1-(3-chloroanilino)-4-phenylphthalazine (MY-5445), and derivatives thereof. The PDE inhibitor used in the present application may be, for example, IBMX. The concentration of the PDE inhibitor can be appropriately selected by those skilled in the art according to the PDE inhibitor to be used. When the PDE inhibitor is IBMX, its concentration may be 400 nM to 40 mM, 4 µM to 40 mM, or 40 µM to 4 mM, and it is, for example, about 400 µM.

In an embodiment, the medium can further comprise a retinoic acid receptor agonist. The "retinoic acid receptor (RAR) agonist" may be a naturally-occurring retinoid, a chemically-synthesized retinoid, a retinoic acid receptor agonist compound without a retinoid backbone, or a natural product having retinoic acid receptor agonist activity. Examples of the natural retinoid with activity as the RAR agonist include a retinoic acid (stereoisomers of all-trans-retinoic acid (all-trans RA) and 9-cis-retinoic acid (9-cisRA) are known). The chemically-synthesized retinoid is known in the art (e.g., U.S. Patent Nos. 5,234,926, and 4,326,055). Examples of the retinoic acid receptor agonist compound without a retinoid backbone include Am80, AM580 (4-[[5,6,7,8-tetrahydro-5,5,8,8-tetramethyl-2-naphthalenyl]carboxamido]benzoic acid), TTNPB (4-[[E]-2-[5,6,7,8-tetrahydro-5,5,8,8-tetramethyl-2-naphthalenyl]-1-propenyl]benzoic acid), and AC55649 (4'-octyl-[1,1'-biphenyl]-4-carboxylic acid). Examples of the natural product having retinoic acid receptor agonist activity include honokiol, and magnolol (Annual report of Research Institute for Biological Function 9: 55-61, 2009). The RAR agonist used in the present application may be a retinoic acid, AM580, TTNPB, and AC55649, and may be for example, TTNPB. In the present embodiment, the concentration of the retinoic acid receptor agonist may be appropriately selected by those skilled in the art according to the retinoic acid receptor agonist to be used. When the retinoic acid receptor agonist is TTNPB, its concentration may be 0.1 nM to 10 µM, 1 nM to 10 µM, or 10 nM to 1 µM, and it is, for example, about 0.1 µM.

In an embodiment, the medium may further comprise a vasopressin receptor agonist. Examples of the vasopressin receptor agonist include, but not particularly limited to, vasopressin (arginine vasopressin; AVP), desmopressin (1-desamino-8-D-arginine vasopressin; dDAVP), lypressin, terlipressin, ornipressin, and felypressin. The vasopressin receptor agonist used in the present application may be vasopressin or desmopressin, and is, for example, desmopressin. Desmopressin can be commercially available, for example, from Sigma Aldrich. In the present embodiment, the concentration of the vasopressin receptor agonist may be appropriately selected by those skilled in the art according to the vasopressin receptor agonist to be used. When the vasopressin receptor agonist is desmopressin, its concentration may be 0.1 nM to 10 µM, 1 nM to 10 µM, or 10 nM to 1 µM, and it is, for example, about 0.1 µM.

In an embodiment, the medium may further comprise aldosterone. Aldosterone can be commercially available, for example, from Sigma Aldrich. In the present embodiment, the concentration of aldosterone may be 0.01 nM to 1 µM, 0.1 nM to 1 µM, or 1 nM to 0.1 µM, and it is, for example, about 10 nM.

In an embodiment, the medium may further comprise a calcium calmodulin kinase (CaMK) inhibitor. Examples of the CaMK inhibitor include K252a, KN-93, KN-62, AIP, CaM kinase II inhibitor 281-301, Lavendustin C, Rottlerin, ML-7, ML-9, STO-609, W-7, and W-5. The CaMK inhibitor used in the present application may be, for example, K252a. In the present embodiment, the concentration of the CaMK inhibitor may be appropriately selected by those skilled in the art according to the CaMK inhibitor to be used. When the CaMK inhibitor is K252a, its concentration may be 0.1 nM to 10 µM, 1 nM to 10 µM, or 10 nM to 1 µM, and it is, for example, about 0.1 µM.

In an embodiment, the ureteric bud cells or the cells that have been differentiated from the ureteric bud cells may be subjected to air-liquid interface culture. The air-liquid interface culture is performed by, for example, culturing cells on a porous membrane located at an interface between the air and liquid phases. For example, it may be performed by seeding cells on a porous membrane of a cell culture insert, making the upper side an air phase, and supplying a medium from the lower side through the membrane. Commercially available products such as transwell (registered trademark) may be used. Ureteric bud principal cells with the polar maintained may be produced by the air-liquid interface culture.

In another embodiment, the ureteric bud cells or the cells that have been differentiated from the ureteric bud cells may be subjected to adherent culture. In the present application, the term "adherent culture" means that cells are cultured in a state of adhering to a culture substrate, and for example, cultured in a coated culture vessel. Examples of the coating agents include Matrigel (Becton, Dickinson and Company), Synthemax (Corning Inc.), collagen, gelatin, laminin (e.g., laminin-511, laminin-111, and laminin-411), heparan sulfate proteoglycan, entactin, fragments thereof, and a combination thereof. In the present application, the ureteric bud cells may be cultured on a cell culture plate coated with extracellular matrix proteins, such as laminin. A commercially available coating agents may be used, and for example, iMatrix-511 silk may be used.

In an embodiment, the culturing period of the process may be 5 to 50 days, 7 to 30 days, or 10 to 21 days, and it is, for example, about 14 days. In another embodiment, when the ureteric bud cells or the cells that have been differentiated from the ureteric bud cells are cultured in the presence of an ETS family protein, the culturing period of the process may be 1 to 30 days, 2 to 14 days, or 3 to 10 days, and it is, for example, about 4 to 6 days.

The culturing temperature is, but not limited to, about 30 to 40°C, for example, about 37°C, and the culture is performed in an atmosphere of CO₂-containing air, with a CO₂ concentration of, for example, about 2 to 5%.

In an embodiment, the method of the present application includes culturing ureteric bud cells in a medium comprising a TGFβ inhibitor and a Wnt inhibitor, prior to the above process. In other words, in an embodiment, the method of the present application comprises:
(A) culturing ureteric bud cells in a medium comprising a TGFβ inhibitor and a Wnt inhibitor; and
(B) culturing the ureteric bud cells or cells that have been differentiated from the ureteric bud cells obtained in (A) in a medium comprising a TGFβ inhibitor, a Wnt inhibitor, and a phosphodiesterase inhibitor. Here, the step (B) corresponds to the above process. In the step (A), the ureteric bud cells may be subjected to adherent culture.

In the step (A), as the TGFβ inhibitor, the above TGFβ inhibitors may be used. The concentration of the TGFβ inhibitor may be appropriately selected by those skilled in the art according to the TGFβ inhibitor to be used. When the TGFβ inhibitor is A83-01, its concentration may be 1 nM to 100 µM, 10 nM to 100 µM, or 100 nM to 10 µM, and it is, for example, about 1 µM.

In the step (A), as the Wnt inhibitor, the above Wnt inhibitors may be used. The concentration of the Wnt inhibitor may be appropriately selected by those skilled in the art according to the Wnt inhibitor to be used. When the Wnt inhibitor is IWR-1, its concentration may be 1 nM to 100 µM, 10 nM to 100 µM, or 100 nM to 10 µM, and it is, for example, about 1 µM.

In the step (A), the medium may further comprise a retinoic acid receptor agonist. As the retinoic acid receptor agonist, the above retinoic acid receptor agonists may be used. The concentration of the retinoic acid receptor agonist may be appropriately selected by those skilled in the art according to the retinoic acid receptor agonist to be used. When the retinoic acid receptor agonist is TTNPB, its concentration may be 0.1 nM to 10 µM, 1 nM to 10 µM, or 10 nM to 1 µM, and it is, for example, about 0.1 µM.

The culturing period in the step (A) is not particularly limited, and is, for example, 1 to 30 days. In an embodiment, the culturing period in the step (A) may be 1 to 10 days, 1 to 7 days, or 1 to 5 days, and it is, for example, about 2 to 3 days. In another embodiment, the culturing period in the step (A) may be 2 to 21 days, 3 to 14 days, or 5 to 10 days, and it is, for example, about 7 days.

In the step (B), the cells differentiated from the ureteric bud cells may be any cells generated before differentiating into renal collecting duct principal cells, and, for example, may be renal collecting duct progenitor cells. When the culturing period in the step (A) is 2 to 21 days, 3 to 14 days, 5 to 10 days, or about 7 days, the cells differentiated from the ureteric bud cells may be renal collecting duct progenitor cells.

The culturing temperature in the step (A) is, but not limited to, about 30 to 40°C, for example, about 37°C, and the culture is performed in an atmosphere of CO₂-containing air, with a CO₂ concentration of, for example, about 2 to 5%.

### Cell Population Comprising Renal Collecting Duct Principal Cells

The present application also provides a cell population comprising 40% or more renal collecting duct principal cells. The cell population comprising 40% or more renal collecting duct principal cells may be produced by the method of the present application. In an embodiment, the cell population comprising 40% or more renal collecting duct principal cells comprises 40% or more, 50% or more, 60% or more, 70% or more, 80% or more, 85% or more, 90% or more, 95% or more, or 99% or more renal collecting duct principal cells. It may be confirmed that the cell population of the present application comprises renal collecting duct principal cells, by the expression of the renal collecting duct principal cell markers described above. It may be confirmed that the cell population of the present application comprises 40% or more renal collecting duct principal cells, by flow cytometry, for example, fluorescence-activated cell sorting (FACS).

In this embodiment, animals from which renal collecting duct principal cells are derived are not limited, and examples thereof include mammals such as a mouse, a rat, a hamster, a guinea pig, a cow, a horse, a pig, a sheep, a monkey, an orangutan, a chimpanzee, a dog, a cat, a bird, and a human, preferably primates, and more preferably a human.

### Method for Producing Renal Collecting Duct Principal Cell-Containing Organoid

The present application also provides a method for producing a renal collecting duct principal cell-containing organoid, including culturing a ureteric bud organoid or an organoid that has been differentiated from a ureteric bud organoid in a medium comprising a TGFβ inhibitor, a Wnt inhibitor, and a phosphodiesterase inhibitor.

In the present application, the renal collecting duct principal cell-containing organoid refers to a self-assembled structure containing renal collecting duct principal cells. The size of the renal collecting duct principal cell-containing organoid is, for example, about 10 to 1000 µm. In an embodiment, the renal collecting duct principal cell-containing organoid comprises 40% or more, 50% or more, 60% or more, 70% or more, 80% or more, 85% or more, 90% or more, 95% or more, or 99% or more renal collecting duct principal cells. It may be confirmed that the renal collecting duct principal cell-containing organoid comprises renal collecting duct principal cells, by the expression of the renal collecting duct principal cell markers, such as AQP2, AQP3, AQP4, and AVPR2. The expression of the renal collecting duct principal cell markers may be visually confirmed under a microscope by immunostaining, or may be confirmed by flow cytometry or fluorescence-activated cell sorting (FACS).

In the present application, animals from which ureteric bud organoids are derived are not limited, and examples thereof include mammals such as a mouse, a rat, a hamster, a guinea pig, a cow, a horse, a pig, a sheep, a monkey, an orangutan, a chimpanzee, a dog, a cat, a bird, and a human, preferably primates, and more preferably a human. The size of the ureteric bud organoid is, for example, about 10 to 1000 µm. Whether there is a ureteric bud organoid may be confirmed by the expression of markers, such as GATA3, RET, WNT11, LHX1, SOX9, and very low-density lipoprotein receptor (VLDL-R), uptake of the very low density lipoprotein (VLDL), or the like. In an embodiment, a ureteric bud organoid is cultured in a medium comprising a TGFβ inhibitor, a Wnt inhibitor, and a phosphodiesterase inhibitor.

The organoid differentiated from the ureteric bud organoid may be any organoid prior to differentiation into a renal collecting duct principal cell-containing organoid, and, for example, may be a renal collecting duct progenitor cell-containing organoid. The renal collecting duct progenitor cell-containing organoid refers to a self-assembled structure containing renal collecting duct progenitor cells. The size of the renal collecting duct progenitor cell-containing organoid is, for example, about 10 to 1000 µm. Whether there is a renal collecting duct progenitor cell-containing organoid may be confirmed by the expression of markers, such as AQP2. In an embodiment, the renal collecting duct principal cell-containing organoid differentiated from the ureteric bud organoid is cultured in a medium comprising a TGFβ inhibitor, a Wnt inhibitor, and a phosphodiesterase inhibitor.

The ureteric bud organoid may be those differentiated from pluripotent stem cells. Methods for differentiating pluripotent stem cells to induce ureteric bud organoids are known, and any known method may be used. For example, the methods described above may be used. In an embodiment, the pluripotent stem cells are iPS cells.

In an embodiment, the ureteric bud organoid or the organoid that has been differentiated from the ureteric bud organoid is cultured in the presence of an ETS family protein. In this embodiment, the ETS family protein may be added in a medium, or cells comprised in the ureteric bud organoid or the organoid that has been differentiated from the ureteric bud organoid may express an ETS family protein. As the ETS family protein, the ETS family proteins described above may be used. When the ETS family protein is added in a medium, the concentration of the ETS family protein may be appropriately selected by those skilled in the art according to the ETS family protein to be used. When the ETS family protein is ELF5, its concentration is, for example, 1 nM to 1 mM.

In an embodiment, the cells comprised in the ureteric bud organoid or the organoid that has been differentiated from the ureteric bud organoid express an ETS family protein. In the present application, the cells that express the ETS family protein may be, for example, cells in which an ETS family gene has been introduced. The procedures to introduce a gene are described above.

In an embodiment, the cells that express the ETS family protein may transiently express the ETS family protein. The transient expression of the ETS family protein may be performed using the methods described above. In particular, the tetracycline (Tet) gene expression induction system may be preferably used. In an embodiment, the transient expression of the ETS family protein may be controlled by the tetracycline expression induction system, and the medium further comprises doxycycline. In the present embodiment, the concentration of doxycycline may be 4 nM to 400 µmM, 40 nM to 400 µM, or 400 nM to 40 µM, and it is, for example, about 4 µM.

The introduced ETS family gene may be removed from the cells when expression is no longer necessary. When the gene transfer vector is an episomal vector, the vector spontaneously disappears after culturing the cells for long term. When a PiggyBac vector is used, the gene that was once integrated into the genome can be removed by PiggyBac (trademark) transposase.

In an embodiment, the ureteric bud organoid may be those induced from pluripotent stem cells that express the ETS family protein. The pluripotent stem cells that express the ETS family protein may be any pluripotent stem cells expressing the ETS family protein, and for example, they are pluripotent stem cells in which the ETS family gene has been introduced. The procedures to introduce a gene are described above.

In an embodiment, the pluripotent stem cells that express the ETS family protein may transiently express the ETS family protein. The transient expression of the ETS family protein may be performed using the methods described above. In particular, the tetracycline (Tet) gene expression induction system may be preferably used. For example, the transient expression of the ETS family protein may be controlled by the tetracycline expression induction system.

In this embodiment, as the TGFβ inhibitor, the above TGFβ inhibitors may be used. The concentration of the TGFβ inhibitor may be appropriately selected by those skilled in the art according to the TGFβ inhibitor to be used. When the TGFβ inhibitor is A83-01, its concentration may be 1 nM to 100 µM, 10 nM to 100 µM, or 100 nM to 10 µM, and it is, for example, about 1 µM.

In this embodiment, as the Wnt inhibitor, the above Wnt inhibitors may be used. The concentration of the Wnt inhibitor may be appropriately selected by those skilled in the art according to the Wnt inhibitor to be used. When the Wnt inhibitor is IWR-1, its concentration may be 1 nM to 100 µM, 10 nM to 100 µM, or 100 nM to 10 µM, and it is, for example, about 1 µM.

In this embodiment, as the PDE inhibitor, the above PDE inhibitors may be used. The concentration of the PDE inhibitor may be appropriately selected by those skilled in the art according to the PDE inhibitor to be used. When the PDE inhibitor is IBMX, its concentration may be 400 nM to 40 mM, 4 µM to 40 mM, or 40 µM to 4 mM, and it is, for example, about 400 µM.

In an embodiment, the medium may further comprise a retinoic acid receptor agonist. As the retinoic acid receptor agonist, the above retinoic acid receptor agonists may be used. The concentration of the retinoic acid receptor agonist may be appropriately selected by those skilled in the art according to the retinoic acid receptor agonist to be used. When the retinoic acid receptor agonist is TTNPB, its concentration may be 0.1 nM to 10 µM, 1 nM to 10 µM, or 10 nM to 1 µM, and it is, for example, about 0.1 µM.

In an embodiment, the medium may further comprise a vasopressin receptor agonist. As the vasopressin receptor agonist, the above vasopressin receptor agonists may be used. The concentration of the vasopressin receptor agonist may be appropriately selected by those skilled in the art according to the vasopressin receptor agonist to be used. When the vasopressin receptor agonist is desmopressin, its concentration may be 0.1 nM to 10 µM, 1 nM to 10 µM, or 10 nM to 1 µM, and it is, for example, about 0.1 µM.

In an embodiment, the medium may further comprise aldosterone. The concentration of aldosterone may be 0.01 nM to 1 µM, 0.1 nM to 1 µM, or 1 nM to 0.1 µM, and it is, for example, about 10 nM.

In an embodiment, the medium may further comprise a CaMK inhibitor. As the CaMK inhibitor, the above CaMK inhibitors may be used. The concentration of the CaMK inhibitor may be appropriately selected by those skilled in the art according to the CaMK inhibitor to be used. When the CaMK inhibitor is K252a, its concentration may be 0.1 nM to 10 µM, 1 nM to 10 µM, or 10 nM to 1 µM, and it is, for example, about 0.1 µM.

In an embodiment, the culturing period of the process may be 5 to 50 days, 7 to 30 days, or 10 to 21 days, and it is, for example, about 14 days. In another embodiment, when the ureteric bud organoid or the organoid that has been differentiated from the ureteric bud organoid is cultured in the presence of the ETS family protein, the culturing period of the process may be 1 to 30 days, 2 to 14 days, or 4 to 8 days, and it is, for example, about 6 days.

In this embodiment, the ureteric bud organoid or the organoid that has been differentiated from the ureteric bud organoid may be subjected to suspension culture. In the present application, the term "suspension culture" means culturing cells in a state without adhering to a culture dish. Although not particularly limited, it may be performed using those that have not been artificially treated for the purpose of improving adhesiveness to cells (e.g., coating treatment with extracellular matrix), or those that have been artificially treated to suppress adhesiveness (e.g., coating treatment with a polymer of polyhydroxyethyl methacrylate (poly-HEMA) or 2-methacryloyloxyethyl phosphorylcholine (Lipidure)). For example, commercially available products such as a low adhesion 96-well plate (Sumitomo Bakelite Co., Ltd.), and 35 mm low adhesion dish (Sumitomo Bakelite Co., Ltd.) may be used.

The culturing temperature is, but not limited to, about 30 to 40°C, for example, about 37°C, and the culture is performed in an atmosphere of CO₂-containing air, with a CO₂ concentration of, for example, about 2 to 5%.

In an embodiment, the method of the present application includes culturing a ureteric bud organoid in a medium comprising a TGFβ inhibitor and a Wnt inhibitor, prior to the above process. In other words, in an embodiment, the method of the present application includes:
(A) culturing a ureteric bud organoid in a medium comprising a TGFβ inhibitor, and a Wnt inhibitor; and
(B) culturing the ureteric bud organoid or an organoid that has been differentiated from the ureteric bud organoid obtained in (A) in a medium comprising a TGFβ inhibitor, a Wnt inhibitor, and a phosphodiesterase inhibitor. Here, the step (B) corresponds to the above process. In the step (A), the ureteric bud organoid may be subjected to suspension culture.

In the step (A), as the TGFβ inhibitor, the above TGFβ inhibitors may be used. The concentration of the TGFβ inhibitor may be appropriately selected by those skilled in the art according to the TGFβ inhibitor to be used. When the TGFβ inhibitor is A83-01, its concentration may be 1 nM to 100 µM, 10 nM to 100 µM, or 100 nM to 10 µM, and it is, for example, about 1 µM.

In the step (A), as the Wnt inhibitor, the above Wnt inhibitors may be used. The concentration of the Wnt inhibitor may be appropriately selected by those skilled in the art according to the Wnt inhibitor to be used. When the Wnt inhibitor is IWR-1, its concentration may be 1 nM to 100 µM, 10 nM to 100 µM, or 100 nM to 10 µM, and it is, for example, about 1 µM.

In the step (A), the medium may further comprise a retinoic acid receptor agonist. As the retinoic acid receptor agonist, the above retinoic acid receptor agonists may be used. The concentration of the retinoic acid receptor agonist may be appropriately selected by those skilled in the art according to the retinoic acid receptor agonist to be used. When the retinoic acid receptor agonist is TTNPB, its concentration may be 0.1 nM to 10 µM, 1 nM to 10 µM, or 10 nM to 1 µM, and it is, for example, about 0.1 µM.

The culturing period in the step (A) is not particularly limited, and is, for example, 1 to 50 days. In an embodiment, the culturing period in the step (A) may be 1 to 10 days, 1 to 7 days, or 2 to 4 days, and it is, for example, about 3 days. In another embodiment, the culturing period in the step (A) may be 5 to 40 days, 7 to 30 days, or 10 to 20 days, and it is, for example, about 14 days.

In the step (B), the organoid differentiated from the ureteric bud organoid may be any organoids generated before differentiating into a renal collecting duct principal cell-containing organoid, and, for example, may be a renal collecting duct progenitor cell-containing organoid. When the culturing period in the step (A) is 5 to 40 days, 7 to 30 days, 10 to 20 days, or about 14 days, the organoid differentiated from the ureteric bud organoid may be a renal collecting duct progenitor cell-containing organoid.

The culturing temperature in the step (A) is, but not limited to, about 30 to 40°C, for example, about 37°C, and the culture is performed in an atmosphere of CO₂-containing air, with a CO₂ concentration of, for example, about 2 to 5%.

### Renal Collecting Duct Principal Cell-Containing Organoid

The present application also provides a renal collecting duct principal cell-containing organoid comprising 40% or more renal collecting duct principal cells. The renal collecting duct principal cell-containing organoid comprising 40% or more renal collecting duct principal cells may be produced by, for example, the method of the present application. In an embodiment, the renal collecting duct principal cell-containing organoid comprising 40% or more renal collecting duct principal cells comprises 40% or more, 50% or more, 60% or more, 70% or more, 80% or more, 85% or more, 90% or more, 95% or more, or 99% or more renal collecting duct principal cells. It may be confirmed that the renal collecting duct principal cell-containing organoid of the present application comprises renal collecting duct principal cells, by the expression of the renal collecting duct principal cell markers described above. It may be confirmed that the renal collecting duct principal cell-containing organoid of the present application comprises 40% or more renal collecting duct principal cells, by flow cytometry, for example, fluorescence-activated cell sorting (FACS).

In this embodiment, animals from which renal collecting duct principal cells are derived are not limited, and examples thereof include mammals such as a mouse, a rat, a hamster, a guinea pig, a cow, a horse, a pig, a sheep, a monkey, an orangutan, a chimpanzee, a dog, a cat, a bird, and a human, preferably primates, and more preferably a human.

### Autosomal Dominant Polycystic Kidney Disease (ADPKD)-Specific Ureteric Bud Cells That Express An ETS Family Protein

The present application also provides autosomal dominant polycystic kidney disease (ADPKD)-specific ureteric bud cells that express ETS family proteins.

The autosomal dominant polycystic kidney disease (ADPKD)-specific cells refer to cells derived from ADPKD patients, or cells that have a genetic mutation associated with ADPKD. The genetic mutation associated with ADPKD refers to a mutation that may cause directly or indirectly ADPKD to occur, by causing an ADPKD-associated gene to become malfunction. Examples of the ADPKD-associated genes include PKD1 gene and PKD2 gene, which are responsible genes for ADPKD, as well as a gene associated therewith, and it is for example, PKD1 gene.

In an embodiment, cells that have a genetic mutation associated with ADPKD are those in which an ADPKD-associated gene is knocked out or knocked down. Method of knocking out or knocking down a gene are known, and not particularly limited. Examples of the method of knocking a gene out include homologous recombination techniques, and genome editing techniques using CRISPR systems, TALEN, ZFN, and the like. Examples of the method of knocking a gene down include an antisense method, in which RNA that corresponds to an antisense strand of mRNA is introduced into cells, an RNAi method using, for example, siRNA, shRNA, and microRNA, and the like.

The ADPKD-specific ureteric bud cells are those derived from ADPKD patients, or those that have a genetic mutation associated with ADPKD. In an embodiment, ADPKD-specific ureteric bud cells are those in which PKD1 gene is knocked out. The ureteric bud cells that have a genetic mutation associated with ADPKD may be produced by genetically modifying the ureteric bud cells, or may be induced from the cells that have a genetic mutation associated with ADPKD.

The ADPKD-specific ureteric bud cells may be those induced to differentiate from pluripotent stem cells. For example, ureteric bud cells may be obtained by dissociating ureteric bud organoids induced to differentiate from pluripotent stem cells, into single cells. Methods for inducing ureteric bud organoids to differentiate from pluripotent stem cells are known, and any known method can be used. For example, the methods described above may be used. In an embodiment, the pluripotent stem cells may be ADPKD-specific pluripotent stem cells, and for example, are those in which PKD1 gene is knocked out. In an embodiment, the pluripotent stem cells are iPS cells.

In this embodiment, the autosomal dominant polycystic kidney disease (ADPKD)-specific ureteric bud cells that express an ETS family protein may be pluripotent stem cells expressing the ETS family protein, and for example, they are ADPKD-specific ureteric bud cells in which the ETS family protein gene has been introduced. The procedures to introduce a gene are described above. As the ETS family protein, the ETS family proteins described above may be used.

In an aspect, the autosomal dominant polycystic kidney disease (ADPKD)-specific ureteric bud cells that express an ETS family protein may transiently express the ETS family protein. The transient expression of the ETS family protein may be performed using the methods described above. In particular, the tetracycline (Tet) gene expression induction system may be preferably used.

### Autosomal Dominant Polycystic Kidney Disease (ADPKD)-Specific Ureteric Bud Organoid That Expresses An ETS Family Protein

The present application also provides an autosomal dominant polycystic kidney disease (ADPKD)-specific ureteric bud organoid that expresses an ETS family protein.

The ADPKD-specific ureteric bud organoid is a ureteric bud organoid derived from an ADPKD patient, or one that has a genetic mutation associated with ADPKD. In an embodiment, the ADPKD-specific ureteric bud organoid is one in which PKD1 gene is knocked out. The ureteric bud organoid that has a genetic mutation associated with ADPKD may be produced by genetically modifying the ureteric bud organoid, or may be induced from the cells that have a genetic mutation associated with ADPKD.

The ADPKD-specific ureteric bud organoid may be one induced to differentiate from pluripotent stem cells. Methods for inducing ureteric bud organoids to differentiate from pluripotent stem cells are known, and any known method can be used. For example, the methods described above may be used. In an embodiment, the pluripotent stem cells may be ADPKD-specific pluripotent stem cells, and for example, are those in which PKD1 gene is knocked out. In an embodiment, the pluripotent stem cells are iPS cells.

In this embodiment, the autosomal dominant polycystic kidney disease (ADPKD)-specific ureteric bud organoid that expresses an ETS family protein may be, for example, an ADPKD-specific ureteric bud organoid in which an ETS family gene has been introduced. The procedures to introduce a gene are described above. As the ETS family protein, the ETS family proteins described above may be used.

In an aspect, the autosomal dominant polycystic kidney disease (ADPKD)-specific ureteric bud organoid that expresses the ETS family protein may transiently express the ETS family protein. The transient expression of the ETS family protein may be performed using the methods described above. In particular, the tetracycline (Tet) gene expression induction system may be preferably used.

### Method for Producing Renal Collecting Duct Intercalated Cells

In an aspect of the present application, provided is a method for producing renal collecting duct intercalated cells, including culturing renal collecting duct principal cells in a medium comprising a TGFβ inhibitor, a Wnt inhibitor, a retinoic acid receptor agonist, and a Notch signal inhibitor, in the presence of an ETS family protein. In an embodiment, the renal collecting duct principal cells may be subjected to air-liquid interface culture or planar culture, and may preferably be subjected to air-liquid interface culture.

In the present application, it may be confirmed that the cells are renal collecting duct intercalated cells, by the expression of markers, such as carbonic anhydrase (CA) II, E-CADHERIN, and ATP6V1B. Optionally, it may be confirmed that UPK2, a marker for renal pelvic epithelial cells, is not expressed. It may be visually confirmed that renal collecting duct intercalated cells express a renal collecting duct intercalated cell marker, under a microscope by immunostaining, or it may be confirmed by flow cytometry, for example, fluorescence-activated cell sorting (FACS).

The renal collecting duct principal cells may be produced by the method of the present application. In other words, in an embodiment, the present aspect further includes producing renal collecting duct principal cells by the method of the present application. In addition, the renal collecting duct principal cells may be induced from pluripotent stem cells. In an embodiment, the pluripotent stem cells are iPS cells.

In this embodiment, an ETS family protein may be added in a medium, or the renal collecting duct principal cells may express an ETS family protein. As the ETS family protein, the ETS family proteins described above may be used. When the ETS family protein is added in a medium, the concentration of the ETS family protein may be appropriately selected by those skilled in the art according to the ETS family protein to be used. When the ETS family protein is ELF5, its concentration is, for example, 1 nM to 1 mM.

In an embodiment, the renal collecting duct principal cells express an ETS family protein. In the present application, the cells that express the ETS family protein may be, for example, cells in which the ETS family gene has been introduced. The procedures to introduce a gene are described above.

In an embodiment, the cells that express an ETS family protein may transiently express the ETS family protein. The transient expression of the ETS family protein may be performed using the methods described above. In particular, the tetracycline (Tet) gene expression induction system may be preferably used. In an embodiment, the transient expression of the ETS family protein may be controlled by the tetracycline expression induction system, and the medium further comprises doxycycline. In the present embodiment, the concentration of doxycycline may be 4 nM to 400 µmM, 40 nM to 400 µM, or 400 nM to 40 µM, and it is, for example, about 4 µM.

The introduced ETS family gene may be removed from cells when expression is no longer necessary. When the gene transfer vector is an episomal vector, the vector spontaneously disappears after culturing the cells for long term. When a PiggyBac vector is used, the gene that was once integrated into the genome can be removed by PiggyBac (trademark) transposase.

In an embodiment, the renal collecting duct principal cells may be those induced from pluripotent stem cells that express an ETS family protein. The pluripotent stem cells that express the ETS family may be, for example, pluripotent stem cells in which the ETS family gene has been introduced. The procedures to introduce a gene are described above.

In an embodiment, the pluripotent stem cells that express an ETS family protein may transiently express an ETS family protein. The transient expression of the ETS family protein may be performed using the methods described above. In particular, the tetracycline (Tet) gene expression induction system may be preferably used. For example, the transient expression of the ETS family protein may be controlled by the tetracycline expression induction system.

In this embodiment, as the TGFβ inhibitor, the above TGFβ inhibitors may be used. The concentration of the TGFβ inhibitor may be appropriately selected by those skilled in the art according to the TGFβ inhibitor to be used. When the TGFβ inhibitor is A83-01, its concentration may be 1 nM to 100 µM, 10 nM to 100 µM, or 100 nM to 10 µM, and it is, for example, about 1 µM.

In this embodiment, as the Wnt inhibitor, the above Wnt inhibitors may be used. The concentration of the Wnt inhibitor may be appropriately selected by those skilled in the art according to the Wnt inhibitor to be used. When the Wnt inhibitor is IWR-1, its concentration may be 1 nM to 100 µM, 10 nM to 100 µM, or 100 nM to 10 µM, and it is, for example, about 1 µM.

In this embodiment, as the retinoic acid receptor agonist, the above retinoic acid receptor agonists may be used. The concentration of the retinoic acid receptor agonist may be appropriately selected by those skilled in the art according to the retinoic acid receptor agonist to be used. When the retinoic acid receptor agonist is TTNPB, its concentration may be 0.1 nM to 10 µM, 1 nM to 10 µM, or 10 nM to 1 µM, and it is, for example, about 0.1 µM.

The Notch signal inhibitor is not particularly limited as long as it can suppress the signaling that is mediated by Notch. Examples of the Notch signal inhibitors include DAPT (GSI-IX), DBZ (Dibenzazepine, YO-01027), RO4929097, Semagacestat (LY450139), LY411575, IMR-1, FLI-06, Crenigacestat (LY3039478), and derivatives thereof. The Notch signal inhibitor used in the present application is, for example, DAPT. In this embodiment, the concentration of the Notch signal inhibitor may be appropriately selected by those skilled in the art according to the Notch signal inhibitor to be used. When the Notch signal inhibitor is DAPT, its concentration may be 5 nM to 500 µM, 50 nM to 500 µM, or 500 nM to 50 µM, and it is, for example, about 5 µM.

The culturing period in the process may be 1 to 30 days, 2 to 14 days, or 5 to 10 days, and it is, for example, about 7 days.

The culturing temperature is, but not limited to, about 30 to 40°C, for example, about 37°C, and the culture is performed in an atmosphere of CO₂-containing air, with a CO₂ concentration of, for example, about 2 to 5%.

### Cell Population Comprising Renal Collecting Duct Intercalated Cells

The present application also provides a cell population comprising 30% or more renal collecting duct intercalated cells. The cell population comprising 30% or more renal collecting duct intercalated cells may be produced by, for example, the method of the present application. In an embodiment, the cell population comprising 30% or more renal collecting duct intercalated cells comprises 30% or more, 40% or more, 50% or more, 60% or more, 70% or more, 80% or more, 85% or more, 90% or more, 95% or more, or 99% or more renal collecting duct intercalated cells. It may be confirmed that the cell population of the present application comprises renal collecting duct intercalated cells, by the expression of the renal collecting duct intercalated cell markers described above. It may be confirmed that the cell population of the present application comprises 30% or more renal collecting duct intercalated cells, by flow cytometry, for example, fluorescence-activated cell sorting (FACS).

In this embodiment, animals from which renal collecting duct intercalated cells are derived are not limited, and examples thereof include mammals such as a mouse, a rat, a hamster, a guinea pig, a cow, a horse, a pig, a sheep, a monkey, an orangutan, a chimpanzee, a dog, a cat, a bird, and a human, preferably primates, and more preferably a human.

### Method for Producing Renal Pelvic Epithelial Cells

In an aspect of the present application, provided is a method for producing renal pelvic epithelial cells, including culturing renal collecting duct progenitor cells in a medium comprising a TGFβ inhibitor, a Wnt inhibitor, a retinoic acid receptor agonist, and a Notch signal inhibitor, in the presence of an ETS family protein. In an embodiment, the renal collecting duct progenitor cells may be subjected to air-liquid interface culture or planar culture, and may preferably be subjected to air-liquid interface culture.

In the present application, it may be confirmed that the cells are renal pelvic epithelial cells, by the expression of markers, such as UPK2, E-CADHERIN, and CK8. Optionally, it may be confirmed that ATP6V1B, a renal collecting duct intercalated cell marker, is not expressed. It may be visually confirmed that renal pelvic epithelial cells express a renal pelvic epithelial cell marker, under a microscope by immunostaining, or it may be confirmed by flow cytometry, for example, fluorescence-activated cell sorting (FACS).

The renal collecting duct progenitor cells may be produced by the above method. For example, the renal collecting duct progenitor cells may be induced by a method including culturing ureteric bud cells in a medium comprising a TGFβ inhibitor, and a Wnt inhibitor. Also, the renal collecting duct progenitor cells may be induced from pluripotent stem cells. In an embodiment, the pluripotent stem cells are iPS cells.

In this embodiment, the ETS family protein may be added in a medium, or the renal collecting duct progenitor cells may express the ETS family protein. As the ETS family protein, the ETS family proteins described above may be used. When the ETS family protein is added in a medium, the concentration of the ETS family protein may be appropriately selected by those skilled in the art according to the ETS family protein to be used. When the ETS family protein is ELF5, its concentration is, for example, 1 nM to 1 mM.

In an embodiment, the renal collecting duct progenitor cells express the ETS family protein. In the present application, the cells that express the ETS family protein may be, for example, cells in which an ETS family gene has been introduced. The procedures to introduce a gene are described above.

In an embodiment, the cells that express a ETS family protein may transiently express the ETS family protein. The transient expression of a ETS family protein may be performed using the methods described above. In particular, the tetracycline (Tet) gene expression induction system may be preferably used. In an embodiment, the transient expression of the ETS family protein may be controlled by the tetracycline expression induction system, and the medium further comprises doxycycline. In the present embodiment, the concentration of doxycycline may be 4 nM to 400 µmM, 40 nM to 400 µM, or 400 nM to 40 µM, and it is, for example, about 4 µM.

The introduced ETS family gene may be removed from cells when expression is no longer necessary. When using an episomal vector, a gene transfer vector spontaneously disappears after long-term culture. In addition, when using a PiggyBac vector, a gene that was once integrated into the genome may be removed by PiggyBac (trademark) transposase.

In an embodiment, the renal collecting duct progenitor cells may be those induced from pluripotent stem cells that express the ETS family protein. The pluripotent stem cells that express the ETS family protein only need to express the ETS family protein, and for example, they are pluripotent stem cells in which an ETS family gene has been introduced. The procedures to introduce a gene are described above.

In an embodiment, the pluripotent stem cells that express the ETS family protein may transiently express the ETS family protein. The transient expression of the ETS family protein may be performed using the methods described above. In particular, the tetracycline (Tet) gene expression induction system may be preferably used. For example, the transient expression of the ETS family protein may be controlled by the tetracycline expression induction system.

In this embodiment, as the TGFβ inhibitor, the above TGFβ inhibitors may be used. The concentration of the TGFβ inhibitor may be appropriately selected by those skilled in the art according to the TGFβ inhibitor to be used. When the TGFβ inhibitor is A83-01, its concentration may be 1 nM to 100 µM, 10 nM to 100 µM, or 100 nM to 10 µM, and it is, for example, about 1 µM.

In this embodiment, as the Wnt inhibitor, the above Wnt inhibitors may be used. The concentration of the Wnt inhibitor may be appropriately selected by those skilled in the art according to the Wnt inhibitor to be used. When the Wnt inhibitor is IWR-1, its concentration may be 1 nM to 100 µM, 10 nM to 100 µM, or 100 nM to 10 µM, and it is, for example, about 1 µM.

In this embodiment, as the retinoic acid receptor agonist, the above retinoic acid receptor agonists may be used. The concentration of the retinoic acid receptor agonist may be appropriately selected by those skilled in the art according to the retinoic acid receptor agonist to be used. When the retinoic acid receptor agonist is TTNPB, its concentration may be 0.1 nM to 10 µM, 1 nM to 10 µM, or 10 nM to 1 µM, and it is, for example, about 0.1 µM.

In this embodiment, as the Notch signal inhibitor, the above Notch signal inhibitors may be used. The concentration of the Notch signal inhibitor may be appropriately selected by those skilled in the art according to the Notch signal inhibitor to be used. When the Notch signal inhibitor is DAPT, its concentration may be 5 nM to 500 µM, 50 nM to 500 µM, or 500 nM to 50 µM, and it is, for example, about 5 µM.

The culturing period in the process may be 1 to 30 days, 2 to 14 days, or 5 to 10 days, and it is, for example, about 7 days.

The culturing temperature is, but not limited to, about 30 to 40°C, for example, about 37°C, and the culture is performed in an atmosphere of CO₂-containing air, with a CO₂ concentration of, for example, about 2 to 5%.

### Cell Population Comprising Renal Pelvic Epithelial Cells

The present application also provides a cell population comprising 40% or more renal pelvic epithelial cells. The cell population comprising 40% or more renal pelvic epithelial cells may be produced by, for example, the method of the present application. In an embodiment, the cell population comprising 40% or more renal pelvic epithelial cells comprises 40% or more, 50% or more, 60% or more, 70% or more, 80% or more, 85% or more, 90% or more, 95% or more, or 99% or more renal pelvic epithelial cells. It may be confirmed that the cell population of the present application comprises renal pelvic epithelial cells, by the expression of the renal pelvic epithelial cell markers described above. It may be confirmed that the cell population of the present application comprises 40% or more renal pelvic epithelial cells, by flow cytometry, for example, fluorescence-activated cell sorting (FACS).

In this embodiment, animals from which renal pelvic epithelial cells are derived are not limited, and examples thereof include mammals such as a mouse, a rat, a hamster, a guinea pig, a cow, a horse, a pig, a sheep, a monkey, an orangutan, a chimpanzee, a dog, a cat, a bird, and a human, preferably primates, and more preferably a human.

### Examples

The present invention will be described in more detail using Examples described below; however, the present invention is not limited by those examples.

### [Materials and Methods]

Details of the reagents used in the Example are shown in Table 1.

**[Table 1]**

| **REAGENT or RESOURCE** | SOURCE | IDENTIFIER |
|---|---|---|
| [deamino-Cys1, D-Arg8]-Vasopressin acetate salt hydrate ≥95% (dDAVP) | Sigma | V1005-.1MG |
| 3-Isobutyl-1-methylxanthine ≥99%), (IBMX) | Sigma | I5879-100MG |
| A83-01 | WAKO | 035-24113 |
| Accutase | Innovative Cell Technologies | AT104 |
| Anti-AVPR2, Rabbit-Poly | AAB | HPA046678 |
| B-27 Supplement minus vitamin A | Thermo Fisher Scientific | 12587001 |
| DMEM/F-12, GlutaMAX+B4:B32 | Thermo Fisher Scientific | 10565042 |
| Donkey anti-Goat IgG- Alexa Fluor 488 | Thermo Fisher Scientific | A11055 |
| Donkey anti-Goat IgG- Alexa Fluor 546 | Thermo Fisher Scientific | A11056 |
| Donkey anti-Goat IgG- Alexa Fluor 647 | Thermo Fisher Scientific | A21447 |
| Donkey anti-Mouse IgG- Alexa Fluor 488 | Thermo Fisher Scientific | A21202 |
| Donkey anti-Mouse IgG- Alexa Fluor 546 | Thermo Fisher Scientific | A10036 |
| Donkey anti-Mouse IgG- Alexa Fluor 647 | Thermo Fisher Scientific | A31571 |
| Donkey anti-Rabbit IgG- Alexa Fluor 488 | Thermo Fisher Scientific | A21206 |
| Donkey anti-Rabbit IgG- Alexa Fluor 546 | Thermo Fisher Scientific | A10040 |
| Donkey anti-Rabbit IgG- Alexa Fluor 647 | Thermo Fisher Scientific | A31573 |
| Doxycycline hyclate | LKT Labs | D5897 |
| ELF5 Polyclonal Antibody | Thermo Fisher scientific | 720380 |
| FBS | biosera | FB-1285/500 |
| iMatrix-511 silk | Nippi | 892021 |
| Hoechst 33342 | Thermo Fisher Scientific | H1399 |
| IWR-1 | MERCK | I0161-5MG |
| K-252a | Sigma | K1639-100UG |
| Mouse anti-AQP2 | Santa Cruz | sc-515770; |
| Mouse anti-E-CADHERIN | R&D | AF648; |
| SCNN1B Polyclonal Antibody | Thermo Fisher scientific | PA5-28909 |
| tolvaptan | Axon Medchem | Axon1591 |
| TTNPB | Santa Cruz | sc-203303 |
| Y-27632 | Wako | 034-24024 |
| aldosterone | Sigma | A9477-5MG |
| vasopressin acetate | Sigma | V9879-1 MG |
| anti-ATP6V1 B1 | Santa Cruz | SC-55544 |
| anti-UPK2 | Novus Biolohicals | NBP2-33389 |
| Benzamil | WAKO | 3380/10 |
| DAPT | R&D | 26634/10 |
| Mouse anti-CK8 | Abcam | ab9023 |

### Method for Producing Tet-On-ELF5-1231A3 iPS Cells

The sequence of ELF5 was cloned from human embryonic kidney tissue cDNA. An entry vector was prepared using pENTR (trademark)/D-TOPO (registered trademark) Cloning Kit (Thermo Fisher Scientific, Inc.). The sequence of ELF5 was inserted into an All-in-one PiggyBac vector (S. Kim et al., (2015) Methods in Molecular Biology) using Gateway (registered trademark) LR Clonase (trademark) II Enzyme Mix (Thermo Fisher Scientific, Inc.). The vector was introduced into undifferentiated iPS cells of 1231A3 strain by electroporation, and cells obtained by ampicillin drug selection were used as the Tet-On-ELF5-1231A3 iPS cells.

### Induction of Renal Collecting Duct Principal Cells: Planar Culture

Ureteric bud organoids on Day 6 were prepared from the Tet-On-ELF5-1231A3 iPS cells using the method previously described (WO2021/066076). In a 1.5 mL tube, 10 ureteric bud organoids were collected and washed twice with 500 µL PBS. 100 µL Accutase was added thereto, and the mixture was incubated for 3 minutes at 37°C, 5% CO₂. The ureteric bud organoids were completely transformed into single cells by pipetting, and 900 µL DMEM/10% PBS was added thereto to count cells.

To a 24-well plate (6-well, 12-well, 48-well or 96-well plate, or 6-well, 12-well, or 24-well transwell plate may also be used), the necessary amount of suspension was collected to achieve 0.25 to 1.0 × 10⁵ cells/cm², and centrifuged at 300 G for 5 minutes. The cell count was preferably 1 × 10⁵ cells/cm². After removing the supernatant, cells were resuspended in a medium comprising DMEM/F12 GlutaMAX + B-27 Supplement minus vitamin A having 10% FBS, 1 µM A83-01, and 1 µM IWR-1 (renal collecting duct progenitor cell-induction medium) added thereto, to which 0.125 µg/cm² iMatrix-511 silk and 10 µM Y-27632 were added, and the suspension was adjusted and seeded so that each well contains 500 µL of the suspension. 0.1 µM TTNPB may be further added the medium.

2 or 3 days later, the medium was washed with 500 µL PBS, and then replaced with a DMEM/F12 GlutaMAX + B-27 Supplement minus vitamin A medium comprising 10% FBS, 1 µM A83-01, 1 µM IWR-1, 4 µM doxycycline, and 400 µM IBMX (renal collecting duct principal cell-induction medium). The medium may further comprise 0.1 µM TTNPB, 100 nM desmopressin (dDAVP), 10 nM aldosterone, and 100 nM K252a, and preferably a medium comprising 3 agents of TTNPB, dDAVP, and aldosterone is suitable. Thereafter, the medium was replaced once every 2 days, and the induction into the principal cells was completed on Day 4.

### Induction of Renal Collecting Duct Principal Cells: Transwell Culture

Ureteric bud organoids on Day 6 were prepared from the Tet-On-ELF5-1231A3 iPS cells using the method previously described (WO2021/066076). In a 1.5 mL tube, 10 ureteric bud organoids were collected and washed twice with 500 µL PBS. 100 µL Accutase was added thereto, and the mixture was incubated for 3 minutes at 37°C, 5% CO₂. The ureteric bud organoids were completely transformed into single cells by pipetting, and 900 µL DMEM/10% PBS was added thereto to count cells.

To evaluate the apical-basal polarity, the cells were seeded on a Transwell 12-well plate (Corning Inc., 3460). Specifically, the necessary amount of suspension was collected to achieve 0.5 to 2 × 10⁵ cells/cm², and centrifuged at 300 G for 5 minutes. The cell count was preferably 1 × 10⁵ cells/cm². After removing the supernatant, cells were resuspended in the renal collecting duct progenitor cell-induction medium, to which 0.125 µg/cm² iMatrix-511 silk and 10 µM Y-27632 were added, and the suspension was adjusted and seeded in the upper chamber so that each well contains 300 µL of the suspension. 0.1 µM TTNPB may be further added the medium.

2 or 3 days later, the medium in the upper chamber was removed, and 300 µL renal collecting duct principal cell-induction medium was placed in the lower chamber. The medium may further comprise 0.1 µM TTNPB, 100 nM dDAVP, 10 nM aldosterone, and 100 nM K252a, and preferably a medium comprising 3 agents of TTNPB, dDAVP, and aldosterone is suitable. Thereafter, the medium was replaced once every 2 days, and the induction into the principal cells having the apical-basal polarity was completed on Day 4.

### Preparation of Renal Collecting Duct Principal Cell-Containing Organoid

Ureteric bud organoids on Day 6 were prepared from the Tet-On-ELF5-1231A3 iPS cells using the method previously described (WO2021/066076). The medium was removed and replaced with the renal collecting duct progenitor cell-induction medium of 100 µL/well. 0.1 µM TTNPB may be further added the medium.

3 days later, the medium was replaced with the renal collecting duct principal cell-induction medium of 100 µL/well. The medium may further comprise 0.1 µM TTNPB, 100 nM dDAVP, 10 nM aldosterone, and 100 nM K252a, and preferably a medium comprising 3 agents of TTNPB, dDAVP, and aldosterone is suitable. The medium was replaced once every 2 days, and the preparation of principal cell-containing organoids was completed on Day 6.

### Induction of Renal Collecting Duct Intercalated Cells

Renal collecting duct principal cells were induced from the Tet-On-ELF5-1231A3 iPS cells by the above-mentioned culturing method using a transwell. For the renal collecting duct principal cells on Days 7-10, the medium in the lower chamber was aspirated and washed with 300 µL PBS. 300 µL renal collecting duct progenitor cell-induction medium comprising 0.1 µM TTNPB, 5 µM DAPT, and 4 µM doxycycline was added thereto. The medium was replaced once every 2 days, and the preparation of renal collecting duct intercalated cells was completed on Day 7.

### Induction of Renal Pelvic Epithelial Cells

Ureteric bud organoids on Day 6 were prepared from the Tet-On-ELF5-1231A3 iPS cells using the method previously described (WO2021/066076). In a 1.5 mL tube, 10 ureteric bud organoids were collected and washed twice with 500 µL PBS. 100 µL Accutase was added thereto, and the mixture was incubated for 3 minutes at 37°C, 5% CO₂. The ureteric bud organoids were completely transformed into single cells by pipetting, and 900 µL DMEM/10% PBS was added thereto to count cells.

To seed the cells on a Transwell 12-well plate (Corning Inc., 3460), the necessary amount of suspension was collected to achieve 0.5 to 2 × 10⁵ cells/cm², and centrifuged at 300 G for 5 minutes. The cell count was preferably 1 × 10⁵ cells/cm². After removing the supernatant, cells were resuspended in the renal collecting duct progenitor cell-induction medium, to which 0.125 µg/cm² iMatrix-511 silk and 10 µM Y-27632 were added, and the suspension was adjusted and seeded in the upper chamber so that each well contains 300 µL of the suspension. 0.1 µM TTNPB may be further added the medium.

On Day 2 or 3, the medium in the upper chamber was aspirated and washed with 300 µL PBS. 300 µL renal collecting duct progenitor cell-induction medium comprising 0.1 µM TTNPB, 5 µM DAPT, and 4 µM doxycycline was added to the lower chamber. The medium was replaced once every 2 days, and the preparation of renal pelvic epithelial cells was completed on Day 7.

### Induction of Renal Collecting Duct Principal Cells Without Requiring Forcible Expression of ELF5

Ureteric bud organoids on Day 6 were prepared from the Tet-On-ELF5-1231A3 iPS cells using the method previously described (WO2021/066076). In a 1.5 mL tube, 10 ureteric bud organoids were collected and washed twice with 500 µL PBS. 100 µL Accutase was added thereto, and the mixture was incubated for 3 minutes at 37°C, 5% CO₂. The ureteric bud organoids were completely transformed into single cells by pipetting, and 900 µL DMEM/10% PBS was added thereto to count cells.

To seed the cells on a Transwell 12-well plate (Corning Inc., 3460), the necessary amount of suspension was collected to achieve 0.5 to 2 × 10⁵ cells/cm², and centrifuged at 300 G for 5 minutes. The cell count was preferably 1 × 10⁵ cells/cm². After removing the supernatant, cells were resuspended in the renal collecting duct progenitor cell-induction medium, to which 0.125 µg/cm² iMatrix-511 silk and 10 µM Y-27632 were added, and the suspension was adjusted and seeded in the upper chamber so that each well contains 300 µL of the suspension. 0.1 µM TTNPB may be further added the medium.

2 or 3 days later, the medium in the upper chamber was removed, and 300 µL renal collecting duct progenitor cell-induction medium comprising 400 µM IBMX was placed in the lower chamber. The medium may further comprise 0.1 µM TTNPB, 100 nM dDAVP, 10 nM aldosterone, and 100 nM K252a, and preferably a medium comprising 3 agents of TTNPB, dDAVP, and aldosterone is suitable. Thereafter, the medium was replaced once every 2 days, and the induction into the renal collecting duct principal cells was completed by the culture for 10 to 21 days, preferably the culture for 14 days.

### Immunostaining Method

The cultured cells were washed twice with PBS, and then, fixed with 4% PFA at 4°C for 20 minutes. The cells were washed twice with PBS, and then, blocked using 2% normal donkey serum (MERCK)/PBT (PBS/0.25% Triton X-100, Nacalai tesque, Inc.) (blocking solution) at room temperature for 1 hour. Each primary antibody was diluted with the blocking solution at a rate of 1:200, and left to stand with samples at 4°C for 24 hours. After washing twice with 0.25% PBT, each secondary antibody was diluted with the blocking solution at a rate of 1:500, and left to stand with samples at room temperature for 2 hours. The cells were observed using a microscope such as BZX-700 (Keyence Corporation).

### mRNA Measuring Methods

RNA was extracted from the cultured cells using RNeasy mini kit (QIAGEN N.V.), and cDNA was synthesized using ReverTRa Ace (TOYOBO Co., Ltd.). Real-time PCR was performed using SYBR Premix Ex Taq II (TaKaRa Bio Inc.). Measured values standardized by the expression level of β actin were used.

### Measurement of Transepithelial Electrical Resistance (TEER) of Renal Collecting Duct Principal Cells

Renal collecting duct principal cells on Day 7 prepared from the Tet-On-ELF5-1231A3 iPS cells by the above-mentioned culturing method using a transwell were used. The medium in the lower chamber was aspirated to add 300 µL of a fresh renal collecting duct principal cell-induction medium comprising 0.1 µM TTNPB, 100 nM dDAVP, and 10 nM aldosterone. 100 µL DMEM/F12 GlutaMAX + B-27 Supplement minus vitamin A + 10% FBS were added to the upper chamber. TEER was measured using Millicell ERS2 (Millipore) with the sensor tip touching the medium in the upper and lower chambers. Measurements were made three times and the average value was taken as the value at 0h. For the Benzamil addition group, 1 µM Benzamil was added to the lower chamber. Cells were incubated at 37°C, and 5% CO₂ for 24 hours. TEER was measured using Millicell ERS2 with the sensor tip touching the medium in the upper and lower chambers. Measurements were made three times and the average value was taken as the value at 24h.

### [Results]

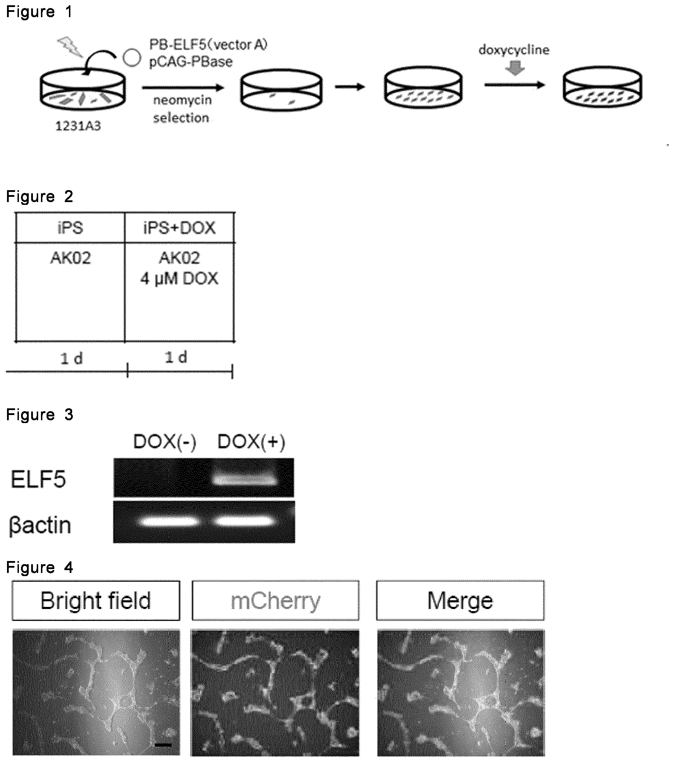

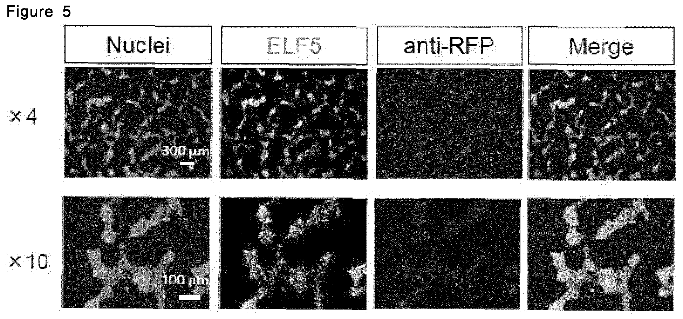

An iPS cell line (Tet-On-ELF5-1231A3) capable of forced expression of ELF5, an upstream gene of renal collecting duct principal cells, was produced (Figure 1). The Tet-On-ELF5-1231A3 iPS cells were cultured in StemFit (registered trademark) AK02 for 1 day, and then, 4 µM doxycycline was added thereto to culture for another 1 day (Figure 2). The results of PCR and immunostaining confirmed that the Tet-On-ELF5-1231A3 iPS cells treated with doxycycline expressed ELF5 and mCherry (Figures 3 to 5).

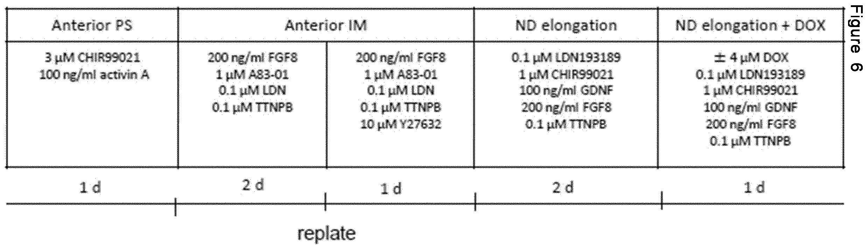

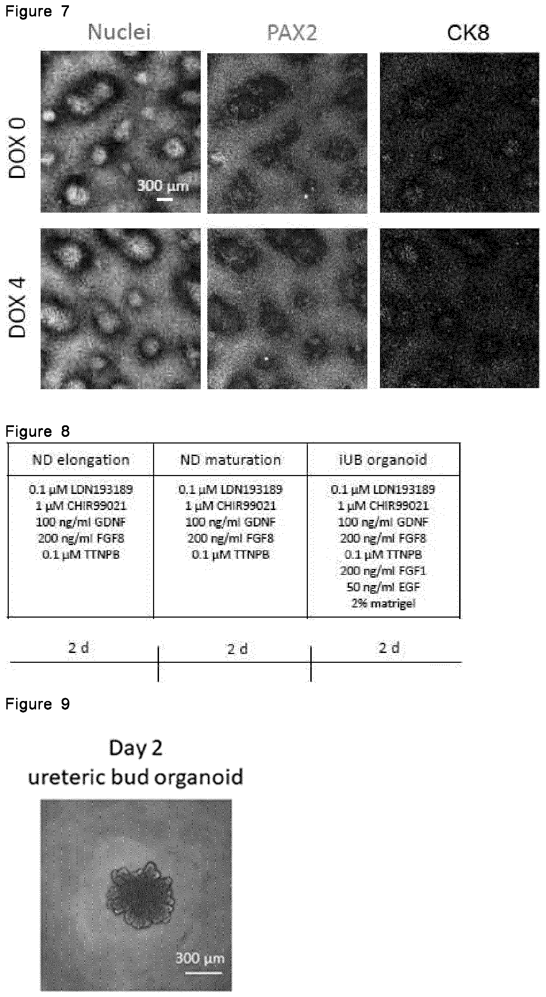

Next, mesonephric duct cells were induced from the Tet-On-ELF5-1231A3 iPS cells according to the method previously described (WO2021/066076, Figure 6). On Day 3 of the mesonephric duct induction, 4 µM doxycycline was added thereto to culture for another 1 day. As a result of immunostaining, the administration of doxycycline did not affect the expression of mesonephric duct marker (Figure 7). In addition, ureteric bud organoids were induced according to the protocol of Figure 8 (Figure 8). It was observed that the established Tet-On-ELF5-1231A3 strain differentiated into ureteric bud organoids (Figure 9).

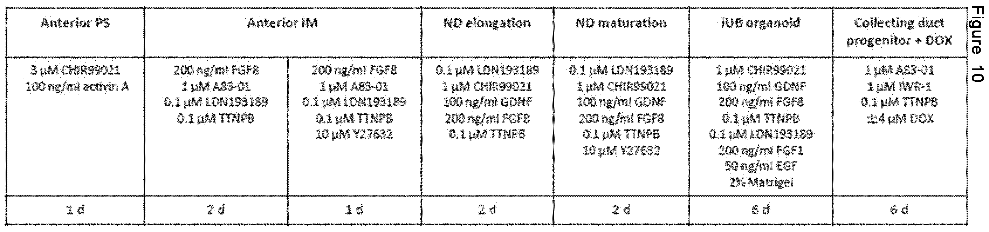

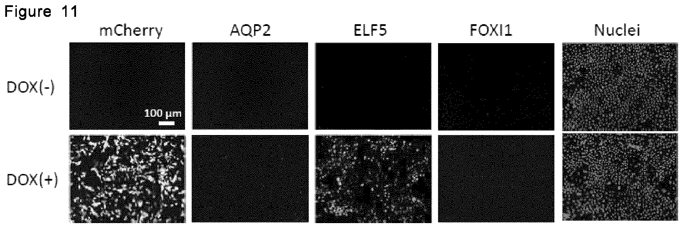

The ureteric bud organoids induced from the Tet-On-ELF5-1231A3 iPS cells were dissociated into single cells as described above, and cultured in a renal collecting duct progenitor cell-induction medium (Figure 10). When 4 µM doxycycline was further added to the renal collecting duct progenitor cell-induction medium, the cultured cells expressed ELF5 and mCherry (Figure 11). The addition of doxycycline did not contribute to the early appearance of AQP2-positive cells.

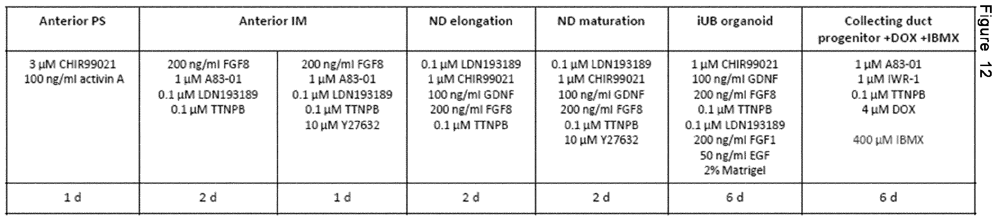

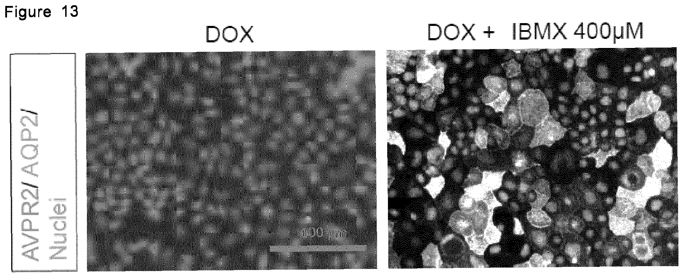

Next, 4 µM doxycycline and 400 µM IBMX were added to the renal collecting duct progenitor cell-induction medium (Figure 12). The cultured cells expressed AVPR2, a renal collecting duct marker, and early expressed AQP2 (Figure 13), suggesting that the further addition of IBMX to the renal collecting duct progenitor cell-induction medium promotes the production of renal collecting duct principal cells.

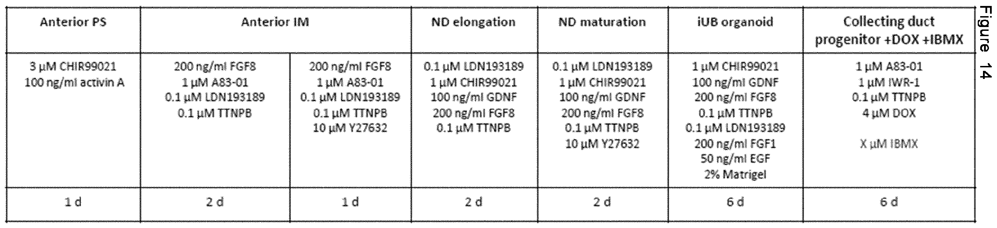

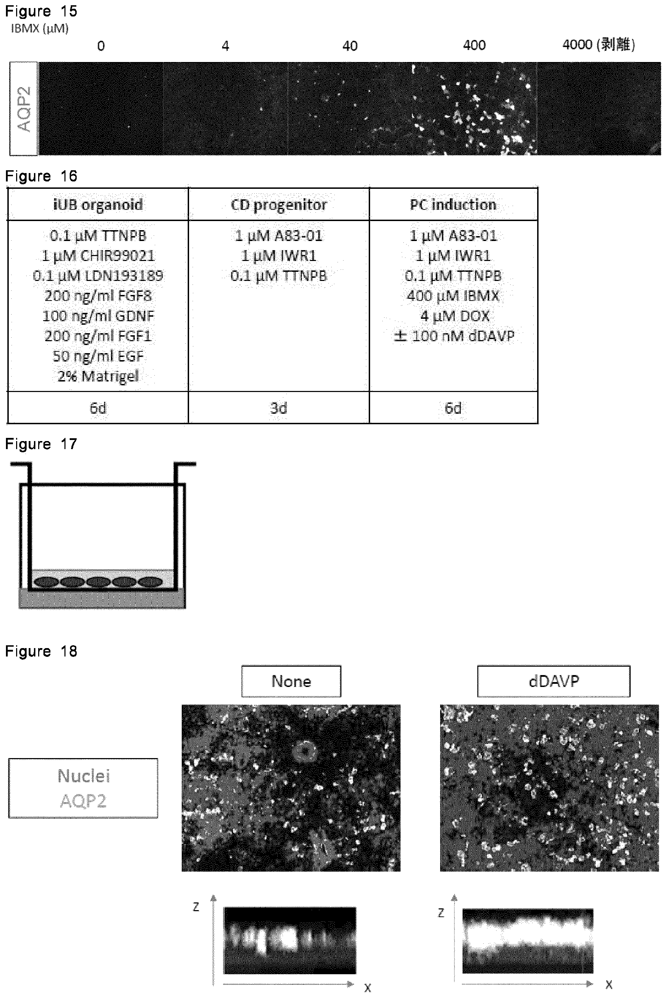

The concentration of IBMX to be added to the renal collecting duct progenitor cell-induction medium was examined (Figure 14). At concentrations up to 400 µM, the number of AQP2-expressing cells concentration-dependently increased; whereas cell detachment was remarkable when 4000 µM IBMX was added (Figure 15).

It has been previously reported that the administration of desmopressin (dDAVP) promotes AQP2 expression and causes apical polarization in mouse renal collecting duct principal cell lines (Ando F. et al., Nat Commun. 2018 Apr 12;9(1):1411). Therefore, renal collecting duct principal cells were induced by transwell culture to evaluate the apical-basal polarity (Figures 16 and 17). When 100 nM dDAVP was further added to the medium in the lower chamber, increase in polarization of AQP2 expression to the contralateral side was observed (Figure 18).

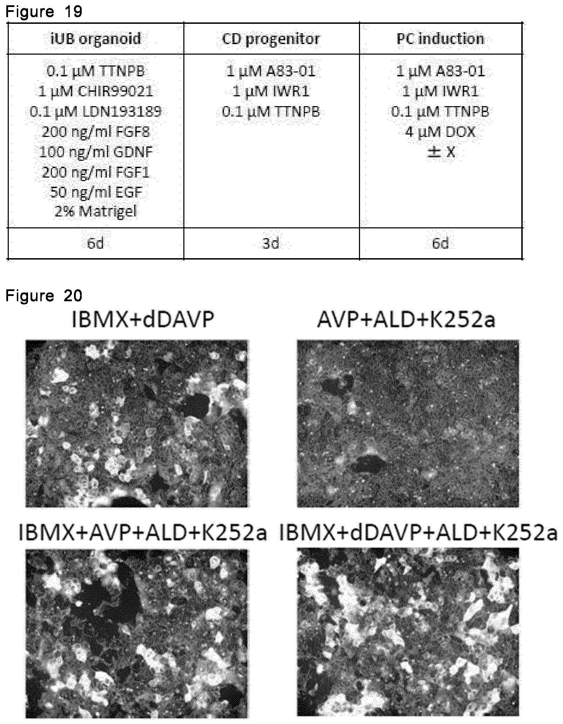

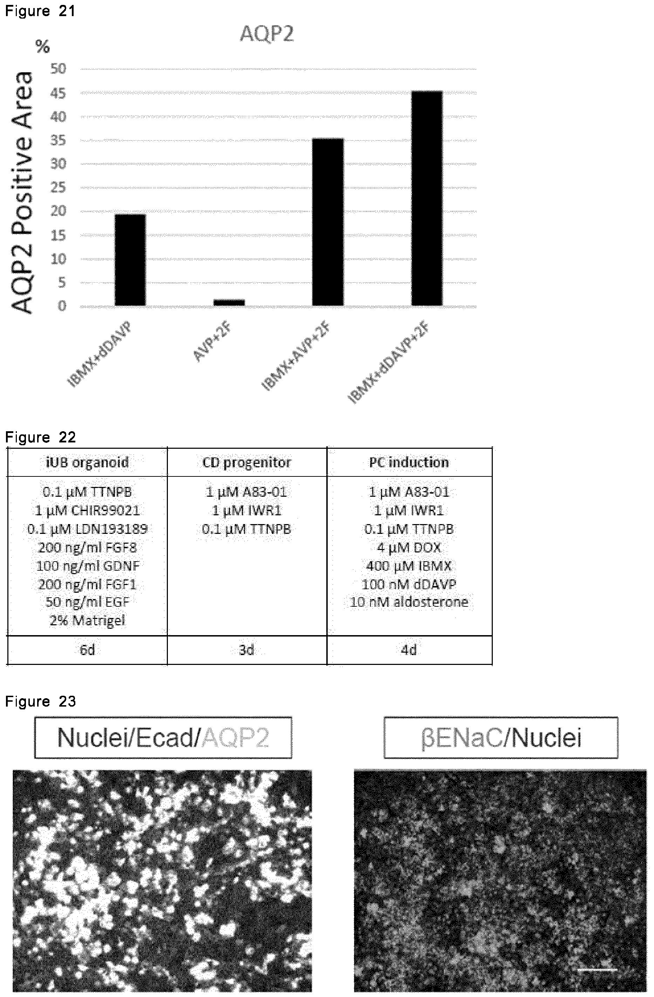

Next, factors effective in inducing renal collecting duct principal cells were examined. Various factors were added to a renal collecting duct principal cell-induction medium excluding IBMX, and renal collecting duct principal cells were induced by planar culture (Figure 19). Although vasopressin acetate (AVP), aldosterone (ALD), and calcium-calmodulin kinase 2 (CaMK2) inhibitor, K252a was added to the medium, the AQP2 expression was not promoted (Figures 20 and 21). On the other hand, when IBMX and dDAVP was added to the medium, or IBMX was added to the medium, either of AVP and dDAVP, ALD, and K252a, the AQP2 expression was promoted, thus suggested that the addition of dDAVP, AVP, ALD, and K252a functioned additively with the addition of IBMX.

Next, to the renal collecting duct principal cell-induction medium, aldosterone, TTNPB, and dDAVP were further added to culture renal collecting duct principal cells (Figure 22). As a result of immunostaining, it was confirmed that renal collecting duct principal cell-like cells that express sodium channels were produced (Figure 23).

Ureteric bud cells were cultured in a renal collecting duct progenitor cell-induction medium comprising TTNPB for 3 days, and further in a renal collecting duct principal cell-induction medium comprising TTNPB, dDAVP, and aldosterone for 4 days to induce renal collecting duct principal cells (Figure 24). The gene expression of the induced renal collecting duct principal cells was compared with that of the cells obtained by culturing ureteric bud cells in a renal collecting duct progenitor cell-induction medium for 7 days (Figure 25). In the renal collecting duct principal cells, the expression of renal collecting duct principal cell-related marker genes (AQP2, AQP3, AVPR2, and SCNN1b) significantly increased. Accordingly, it was confirmed that the induced renal collecting duct principal cells exhibited the properties of renal collecting duct principal cells also at the mRNA level.

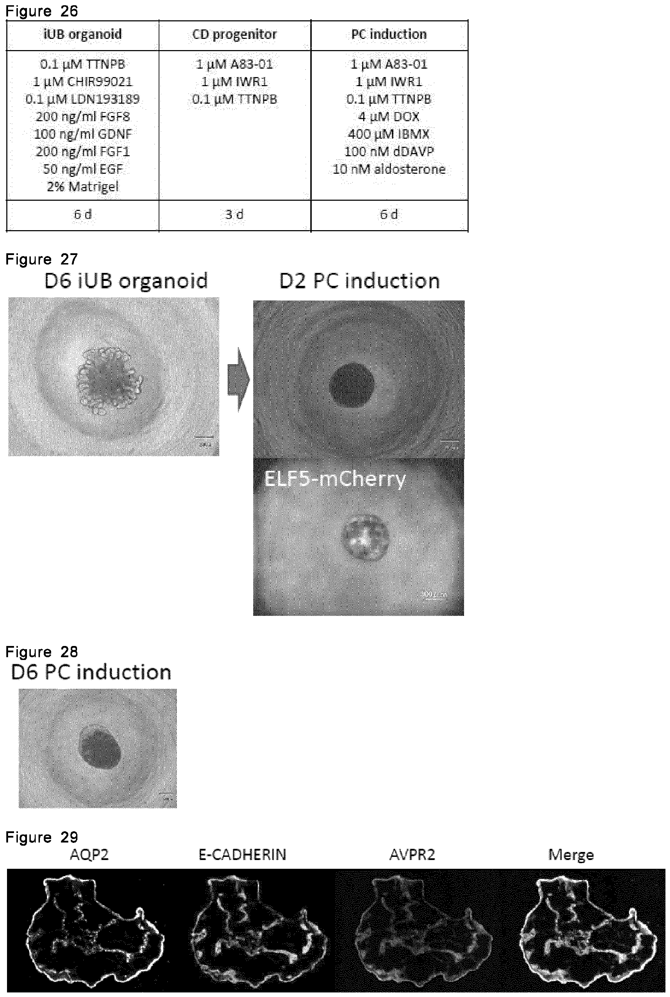

Next, renal collecting duct principal cell-containing organoids were induced from the Tet-On-ELF5-1231A3 iPS cells (Figures 26 to 28). As a result of immunostaining, the renal collecting duct principal cell-containing organoid expressed renal collecting duct principal cell markers, AQP2, E-CADHERIN, and AVPR2 (Figure 29).

The functional evaluation tests were performed on the renal collecting duct principal cell-containing organoids. For the renal collecting duct principal cell-containing organoid on Day 6 of the induction, the medium was replaced with a renal collecting duct principal cell-induction medium, to which 1 µM dDAVP, or with 1 µM dDAVP and 10 µM tolvaptan were added.

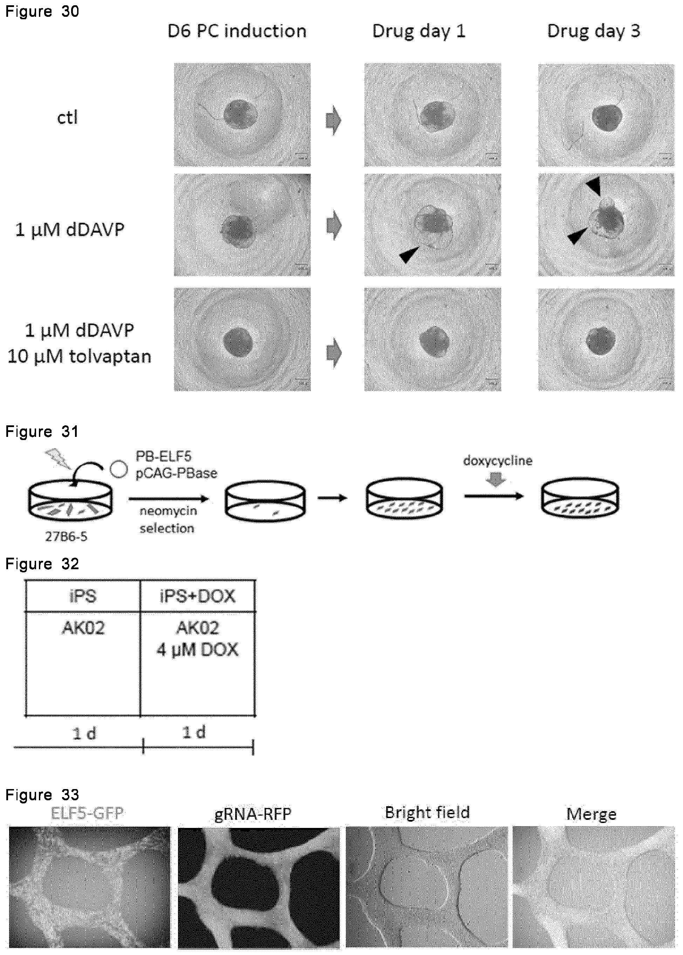

When dDAVP was added to the medium, water uptake was promoted from the next day, and a cystoid structure was formed (Figure 30). When w a vasopressin V2 receptor (V2R) blocker, tolvaptan was added to the medium together with dDAVP, the formation of a cystoid structure was suppressed, thus suggesting that the renal collecting duct principal cell-containing organoid exhibited reactivity to existent agents.

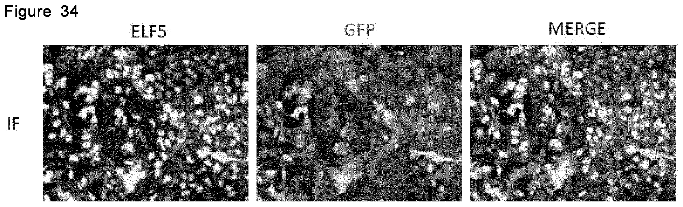

Using PKD1 knockout iPS cells produced by the method previously reported (Ishida K. et al., Sci Rep. 8:310 (2018); Shimizu T. et al., Biochem Biophys Res Commun. 529, 1186-1194 (2020)), PKD1 knockout iPS cell lines capable of forced expression of ELF5 (Tet-On-ELF5 27B6-5-1 to -5 strains) were produced (Figure 31). The cells were cultured in StemFit (registered trademark) AK02 for 1 day, and then, 4 µM doxycycline was added thereto to culture for another 1 day (Figure 32). As a result of immunostaining, it was confirmed that at least two cell lines (Tet-On-ELF5-27B6-5-1 strain and Tet-On-ELF5 27B6-5-2 strain) expressed ELF5 and gRNA (Figures 33 to 34). Figures 33 and 34 show the results of experiments using the Tet-On-ELF5 27B6-5-1 strain.

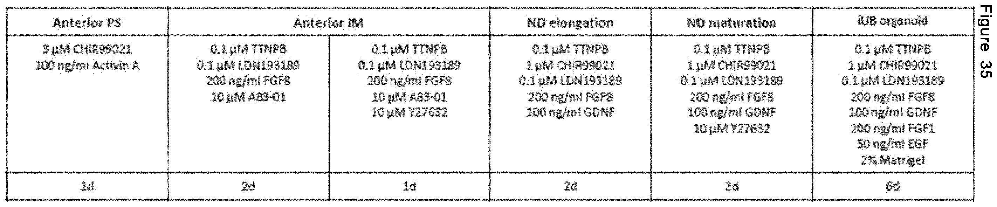

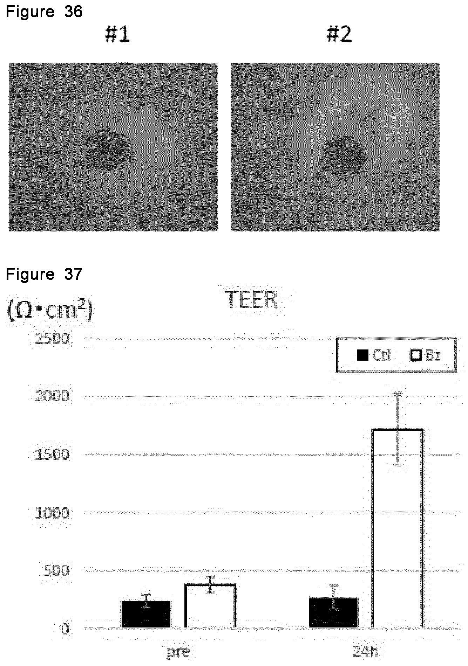

Next, ureteric bud organoids were induced from Tet-On-ELF5 27G6 cells according to the method previously described (WO2021/066076, Figure 35). The budding deformation was observed in the ureteric bud organoid on Day 2 which was induced in 2% Matrigel (Figure 36). Accordingly, the ureteric bud organoid induced from the Tet-On-ELF5 27G6 cells exhibited branching similar to ureteric bud organoids induced from healthy iPS cell lines.

Next, the functional evaluation tests were performed on the renal collecting duct principal cells produced by the method of the present application. The renal collecting duct principal cells are known to enhance transepithelial electrical resistance (TEER) by the addition of sodium channel blockers (e.g., amiloride, and benzamil). Therefore, the Na channels were evaluated by TEER measurement in the renal collecting duct principal cells produced using transwell by the method of the present application.

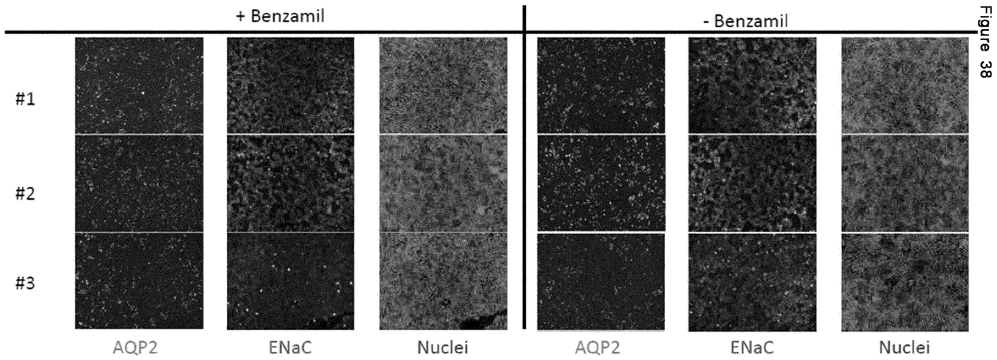

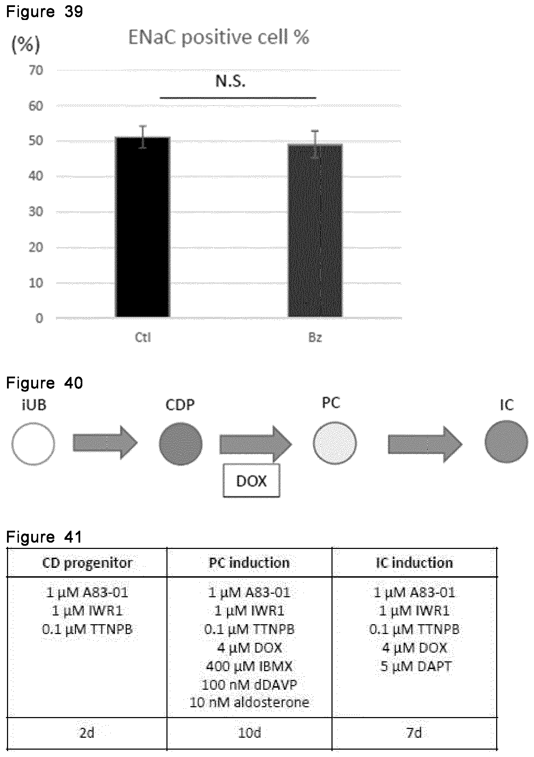

The renal collecting duct principal cells produced by the method of the present application exhibited that TEER enhancement by the addition of benzamil while continuously administering aldosterone (Figure 37). In addition, since ENaC-positive cell rates did not change upon the administration of benzamil, it was confirmed that this fluctuation in TEER was not caused by the change in the differentiation state (Figures 38 and 39).

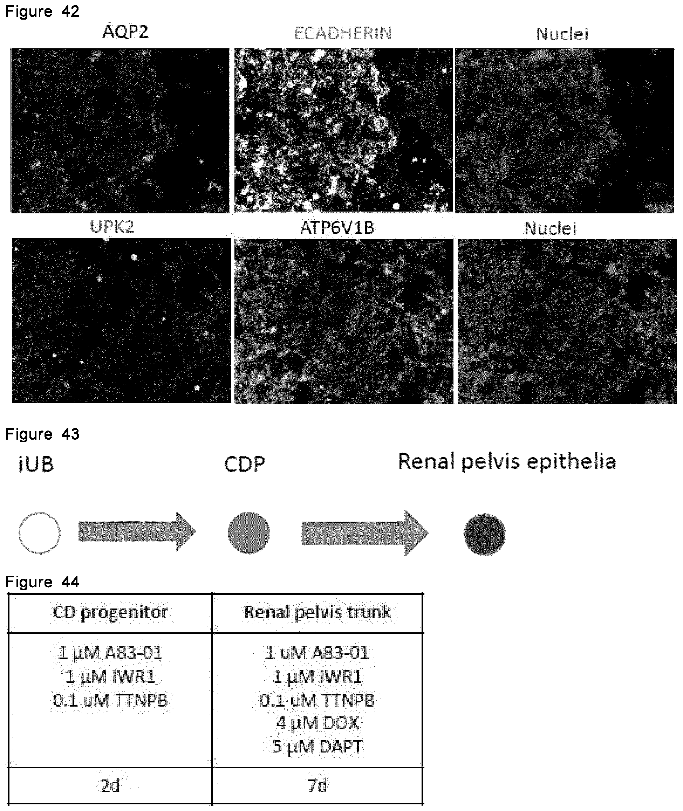

The renal collecting duct intercalated cells were successfully induced to differentiate from the renal collecting duct principal cells (Figures 40 and 41). As a result of immunostaining, AQP2-positive cells decreased in the induced renal collecting duct intercalated cells, and ATP6V1B, a renal collecting duct intercalated cell marker, was positive (Figure 42). In addition, UPK2, a renal pelvic epithelial cell marker, was negative.

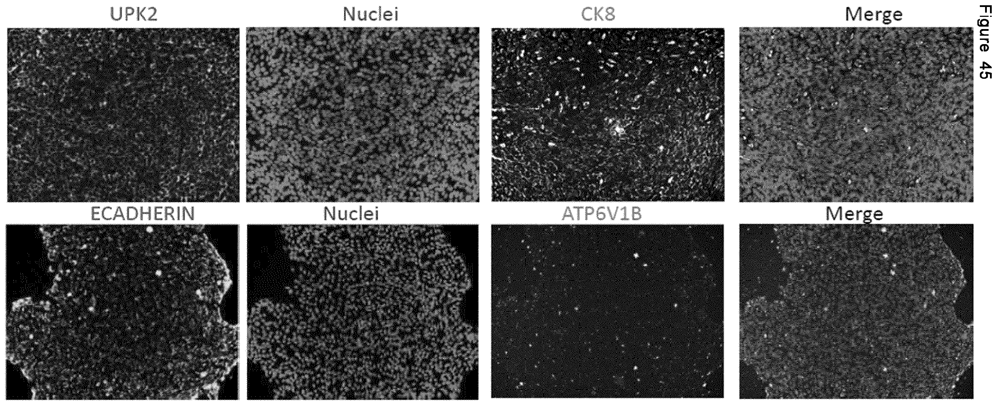

Further, the renal pelvic epithelial cells were successfully induced to differentiate from the renal collecting duct progenitor cells (Figures 43 and 44). As a result of immunostaining, UPK2, a renal pelvic epithelial cell marker, was positive (Figure 45).

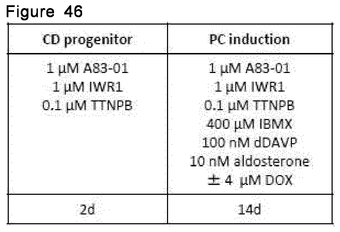

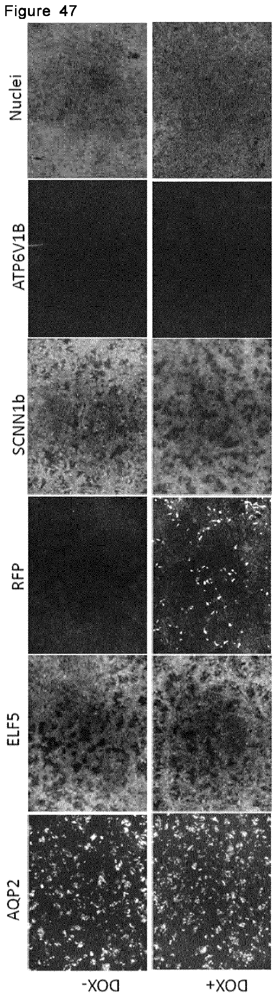

The present inventors also succeeded in inducing renal collecting duct principal cells by extending the culturing period, without forced expression of ELF5. When renal collecting duct progenitor cells were treated on transwell in a renal collecting duct principal cell-induction medium (10% FBS, 1 µM A83-01, 1 µM IWR-1, and 400 µM IBMX) excluding DOX, for 14 days, ELF5 expression was observed without DOX administration. It was therefore indicated that renal collecting duct principal cells may be induced without forced expression of ELF5 due to DOX administration (Figures 46 and 47).

## Claims

1. A method for producing renal collecting duct principal cells, comprising culturing ureteric bud cells or cells that have been differentiated from ureteric bud cells in a medium comprising a TGFβ inhibitor, a Wnt inhibitor, and a phosphodiesterase inhibitor.

2. The method according to claim 1, wherein the ureteric bud cells are cultured in the medium comprising a TGFβ inhibitor, a Wnt inhibitor, and a phosphodiesterase inhibitor.

3. The method according to claim 1, wherein renal collecting duct progenitor cells that have been differentiated from the ureteric bud cells are cultured in the medium comprising a TGFβ inhibitor, a Wnt inhibitor, and a phosphodiesterase inhibitor.

4. The method according to claim 1, comprising:
(A) culturing ureteric bud cells in a medium comprising a TGFβ inhibitor, and a Wnt inhibitor; and
(B) culturing the ureteric bud cells or cells that have been differentiated from the ureteric bud cells obtained in (A) in a medium comprising a TGFβ inhibitor, a Wnt inhibitor, and a phosphodiesterase inhibitor.

5. The method according to any one of claims 1 to 4, wherein the ureteric bud cells or the cells that have been differentiated from the ureteric bud cells are cultured in the presence of an ETS family protein.

6. The method according to claim 5, wherein ureteric bud cells capable of transiently expressing an ETS family protein or cells capable of transiently expressing an ETS family protein that have been differentiated from ureteric bud cells are cultured in the medium comprising a TGFβ inhibitor, a Wnt inhibitor, and a phosphodiesterase inhibitor under conditions where the ETS family protein is expressed.

7. The method according to claim 5 or 6, wherein the ETS family protein is ELF5.

8. The method according to any one of claims 1 to 7, wherein the TGFβ inhibitor is A83-01, the Wnt inhibitor is IWR-1, and the phosphodiesterase inhibitor is IBMX.

9. The method according to any one of claims 1 to 8, wherein the medium further comprises a retinoic acid receptor agonist, a vasopressin receptor agonist, a CaMK inhibitor, and aldosterone.

10. The method according to claim 9, wherein the retinoic acid receptor agonist is TTNPB, the vasopressin receptor agonist is desmopressin, and the CaMK inhibitor is K252a.

11. The method according to any one of claims 1 to 10, wherein the ureteric bud cells are induced from pluripotent stem cells.

12. The method according to claim 11, wherein the pluripotent stem cells are iPS cells.

13. A cell population comprising 40% or more renal collecting duct principal cells.

14. A method for producing a renal collecting duct principal cell-containing organoid, comprising culturing a ureteric bud organoid or an organoid that has been differentiated from a ureteric bud organoid in a medium comprising a TGFβ inhibitor, a Wnt inhibitor, and a phosphodiesterase inhibitor.

15. The method according to claim 14, wherein the ureteric bud organoid is cultured in the medium comprising a TGFβ inhibitor, a Wnt inhibitor, and a phosphodiesterase inhibitor.

16. The method according to claim 14, wherein a renal collecting duct progenitor cell-containing organoid that has been differentiated from the ureteric bud organoid is cultured in the medium comprising a TGFβ inhibitor, a Wnt inhibitor, and a phosphodiesterase inhibitor.

17. The method according to claim 14, comprising:
(A) culturing a ureteric bud organoid in a medium comprising a TGFβ inhibitor, and a Wnt inhibitor; and
(B) culturing the ureteric bud organoid or an organoid that has been differentiated from the ureteric bud organoid obtained in (A) in the medium comprising a TGFβ inhibitor, a Wnt inhibitor, and a phosphodiesterase inhibitor.

18. The method according to any one of claims 14 to 17, wherein cells comprised in the ureteric bud organoid or the organoid that has been differentiated from the ureteric bud organoid is cultured in the presence of an ETS family protein.

19. The method according to claim 18, wherein a ureteric bud organoid comprising cells capable of transiently expressing an ETS family protein or an organoid capable of transiently expressing an ETS family protein that has been differentiated from ureteric bud organoid is cultured in the medium comprising a TGFβ inhibitor, a Wnt inhibitor, and a phosphodiesterase inhibitor under conditions where the ETS family protein is expressed.

20. The method according to claim 18 or 19, wherein the ETS family protein is ELF5.

21. The method according to any one of claims 14 to 20, wherein the TGFβ inhibitor is A83-01, the Wnt inhibitor is IWR-1, and the phosphodiesterase inhibitor is IBMX.

22. The method according to any one of claims 14 to 21, wherein the medium further comprises a retinoic acid receptor agonist, a vasopressin receptor agonist, a CaMK inhibitor, and aldosterone.

23. The method according to claim 22, wherein the retinoic acid receptor agonist is TTNPB, the vasopressin receptor agonist is desmopressin, and the CaMK inhibitor is K252a.

24. The method according to any one of claims 14 to 23, wherein the ureteric bud organoid is induced from pluripotent stem cells.

25. The method according to claim 24, wherein the pluripotent stem cells are iPS cells.

26. A renal collecting duct principal cell-containing organoid comprising 40% or more renal collecting duct principal cells.

27. A method for producing renal collecting duct intercalated cells, comprising culturing renal collecting duct principal cells in a medium comprising a TGFβ inhibitor, a Wnt inhibitor, a retinoic acid receptor agonist, and a Notch signal inhibitor, in the presence of an ETS family protein.

28. The method according to claim 27, wherein the renal collecting duct principal cells express the ETS family protein.

29. The method according to claim 28, wherein renal collecting duct principal cells capable of transiently expressing the ETS family protein are cultured under conditions where the ETS family protein is expressed.

30. The method according to any one of claims 27 to 29, wherein the ETS family protein is ELF5, the TGFβ inhibitor is A83-01, the Wnt inhibitor is IWR-1, the retinoic acid receptor agonist is TTNPB, and the Notch signal inhibitor is DAPT.

31. The method according to any one of claims 27 to 30, further comprising producing renal collecting duct principal cells by the method according to any one of claims 1 to 12.

32. The method according to any one of claims 27 to 31, wherein the renal collecting duct principal cells are induced from pluripotent stem cells.

33. The method according to claim 32, wherein the pluripotent stem cells are iPS cells.

34. A cell population comprising 30% or more renal collecting duct intercalated cells.

35. A method for producing renal pelvic epithelial cells, comprising culturing renal collecting duct progenitor cells in a medium comprising a TGFβ inhibitor, a Wnt inhibitor, a retinoic acid receptor agonist, and a Notch signal inhibitor, in the presence of an ETS family protein.

36. The method according to claim 35, wherein the renal collecting duct progenitor cells express the ETS family protein.

37. The method according to claim 36, wherein renal collecting duct progenitor cells capable of transiently expressing the ETS family protein are cultured under conditions where the ETS family protein is expressed.

38. The method according to any one of claims 35 to 37, wherein the ETS family protein is ELF5, the TGFβ inhibitor is A83-01, the Wnt inhibitor is IWR-1, the retinoic acid receptor agonist is TTNPB, and the Notch signal inhibitor is DAPT.

39. The method according to any one of claims 35 to 38, wherein the renal collecting duct progenitor cells are induced from pluripotent stem cells.

40. The method according to claim 39, wherein the pluripotent stem cells are iPS cells.

41. A cell population comprising 40% or more renal pelvic epithelial cells.
